# EUROPEAN PATENT APPLICATION

(11) **EP 4 183 796 A1**
(43) Date of publication of application: **24.05.2023**
(21) Application number: 21209408.0
(22) Date of filing: 19.11.2021
(51) Int. Cl.: C07K 14/475

(54) **THERMOSTABLE FGF10 POLYPEPTIDE OR FRAGMENT THEREOF USE THEREOF**

(71) Applicant: Enantis s.r.o., 62500 Brno (CZ)
(72) Inventor: CHALOUPKOVA, Radka, 56401 Zamberk (CZ); STEPANKOVA, Veronika, 68401 Slavkov u Brna (CZ); HORACKOVA, Aneta, 69163 Velke Nemcice (CZ); BEDNAR, David, 62500 Brno (CZ); KUTALKOVA, Katerina, 68201 Vyskov (CZ)
(74) Representative: Dvorakova, Martina

(57) **Abstract**

The invention relates to a thermostable FGF10 polypeptide possessing FGF10 activity and having or comprising at least 85% sequence identity to SEQ ID NO:3 that has or comprises an amino acid sequence from Ser69 to Ser208 of SEQ ID NO:1, or to SEQ ID NO:5 that has or comprises an amino acid sequence from Leu40 to Ser208 of SEQ ID NO:1, or the fragments thereof, comprising at least an amino acid substitution L152F. Preferably, any of amino acid substitutions V123I, Q175E, and N181D can be further included. The invention further discloses the use of subjected thermostable FGF10 polypeptides in regenerative medicine or other related medical applications or cosmetics. Further, it discloses a culture medium comprising subjected thermostable FGF10 polypeptides suitable for proliferation and differentiation of the human embryonic stem cells or formation and differentiation of spheroids and organoids.

SEQUENCE LISTING

<110> Enantis s.r.o

<120> Thermostable FGF10 polypeptide or fragment thereof and use thereof

<130> D5055-EP/Ma

<160> 18

<170> BiSSAP 1.3

<210> 1
<211> 208
<212> PRT
<213> Artificial Sequence

<220>
<223> Human FGF10

<400> 1

<210> 2
<211> 37
<212> PRT
<213> Artificial Sequence

<220>
<223> N-terminal signal peptide of human FGF10

<400> 2
Met Trp Lys Trp Ile Leu Thr His Cys Ala Ser Ala Phe Pro His Leu
1               5                   10                  15
Pro Gly Cys Cys Cys Cys Cys Phe Leu Leu Leu Phe Leu Val Ser Ser
            20                  25                  30

Val Pro Val Thr Cys
        35

<210> 3
<211> 140
<212> PRT
<213> Artificial Sequence

<220>
<223> Ser69-Ser208 fragment of SEQ ID NO: 1 + L152F

<400> 3
Ser Tyr Asn His Leu Gln Gly Asp Val Arg Trp Arg Lys Leu Phe Ser
1               5                   10                  15
Phe Thr Lys Tyr Phe Leu Lys Ile Glu Lys Asn Gly Lys Val Ser Gly
            20                  25                  30
Thr Lys Lys Glu Asn Cys Pro Tyr Ser Ile Leu Glu Ile Thr Ser Val
        35                  40                  45
Glu Ile Gly Val Val Ala Val Lys Ala Ile Asn Ser Asn Tyr Tyr Leu
    50                  55                  60
Ala Met Asn Lys Lys Gly Lys Leu Tyr Gly Ser Lys Glu Phe Asn Asn
65                  70                  75                  80
Asp Cys Lys Phe Lys Glu Arg Ile Glu Glu Asn Gly Tyr Asn Thr Tyr
                85                  90                  95
Ala Ser Phe Asn Trp Gln His Asn Gly Arg Gln Met Tyr Val Ala Leu
            100                 105                 110
Asn Gly Lys Gly Ala Pro Arg Arg Gly Gln Lys Thr Arg Arg Lys Asn
        115                 120                 125
Thr Ser Ala His Phe Leu Pro Met Val Val His Ser
    130                 135                 140

<210> 4
<211> 68
<212> PRT
<213> Artificial Sequence

<220>
<223> N-terminal Met1-Arg68 part of SEQ ID NO: 1

<400> 4
Met Trp Lys Trp Ile Leu Thr His Cys Ala Ser Ala Phe Pro His Leu
1               5                   10                  15
Pro Gly Cys Cys Cys Cys Cys Phe Leu Leu Leu Phe Leu Val Ser Ser
            20                  25                  30
Val Pro Val Thr Cys Gln Ala Leu Gly Gln Asp Met Val Ser Pro Glu
        35                  40                  45
Ala Thr Asn Ser Ser Ser Ser Ser Phe Ser Ser Pro Ser Ser Ala Gly
    50                  55                  60
Arg His Val Arg
65

<210> 5
<211> 169
<212> PRT
<213> Artificial Sequence

<220>
<223> Leu40-Ser208 fragment of SEQ ID NO: 1+L152F

<400> 5
Leu Gly Gln Asp Met Val Ser Pro Glu Ala Thr Asn Ser Ser Ser Ser
1               5                   10                  15
Ser Phe Ser Ser Pro Ser Ser Ala Gly Arg His Val Arg Ser Tyr Asn

20                  25                  30
His Leu Gln Gly Asp Val Arg Trp Arg Lys Leu Phe Ser Phe Thr Lys
        35                  40                  45
Tyr Phe Leu Lys Ile Glu Lys Asn Gly Lys Val Ser Gly Thr Lys Lys
    50                  55                  60
Glu Asn Cys Pro Tyr Ser Ile Leu Glu Ile Thr Ser Val Glu Ile Gly
65                  70                  75                  80
Val Val Ala Val Lys Ala Ile Asn Ser Asn Tyr Tyr Leu Ala Met Asn
                85                  90                  95
Lys Lys Gly Lys Leu Tyr Gly Ser Lys Glu Phe Asn Asn Asp Cys Lys
            100                 105                 110
Phe Lys Glu Arg Ile Glu Glu Asn Gly Tyr Asn Thr Tyr Ala Ser Phe
        115                 120                 125
Asn Trp Gln His Asn Gly Arg Gln Met Tyr Val Ala Leu Asn Gly Lys
    130                 135                 140
Gly Ala Pro Arg Arg Gly Gln Lys Thr Arg Arg Lys Asn Thr Ser Ala
145                 150                 155                 160
His Phe Leu Pro Met Val Val His Ser
                165

<210> 6
<211> 39
<212> PRT
<213> Artificial Sequence

<220>
<223> N-terminal Met1-Ala39 part of SEQ ID NO: 1

<400> 6
Met Trp Lys Trp Ile Leu Thr His Cys Ala Ser Ala Phe Pro His Leu
1               5                   10                  15
Pro Gly Cys Cys Cys Cys Cys Phe Leu Leu Leu Phe Leu Val Ser Ser
            20                  25                  30
Val Pro Val Thr Cys Gln Ala
        35

<210> 7
<211> 169
<212> PRT
<213> Artificial Sequence

<220>
<223> Leu40-Ser208 fragment of SEQ ID NO: 1 + L152F+V123I

<400> 7
Leu Gly Gln Asp Met Val Ser Pro Glu Ala Thr Asn Ser Ser Ser Ser
1               5                   10                  15
Ser Phe Ser Ser Pro Ser Ser Ala Gly Arg His Val Arg Ser Tyr Asn
            20                  25                  30
His Leu Gln Gly Asp Val Arg Trp Arg Lys Leu Phe Ser Phe Thr Lys
        35                  40                  45
Tyr Phe Leu Lys Ile Glu Lys Asn Gly Lys Val Ser Gly Thr Lys Lys
    50                  55                  60
Glu Asn Cys Pro Tyr Ser Ile Leu Glu Ile Thr Ser Val Glu Ile Gly
65                  70                  75                  80
Val Val Ala Ile Lys Ala Ile Asn Ser Asn Tyr Tyr Leu Ala Met Asn
                85                  90                  95
Lys Lys Gly Lys Leu Tyr Gly Ser Lys Glu Phe Asn Asn Asp Cys Lys
            100                 105                 110
Phe Lys Glu Arg Ile Glu Glu Asn Gly Tyr Asn Thr Tyr Ala Ser Phe
        115                 120                 125
Asn Trp Gln His Asn Gly Arg Gln Met Tyr Val Ala Leu Asn Gly Lys
    130                 135                 140

Gly Ala Pro Arg Arg Gly Gln Lys Thr Arg Arg Lys Asn Thr Ser Ala
145                 150                 155                 160
His Phe Leu Pro Met Val Val His Ser
                165

<210> 8
<211> 169
<212> PRT
<213> Artificial Sequence

<220>
<223> Leu40-Ser208 fragment of SEQ ID NO: 1 + L152F+Q175E

<400> 8
Leu Gly Gln Asp Met Val Ser Pro Glu Ala Thr Asn Ser Ser Ser Ser
1               5                   10                  15
Ser Phe Ser Ser Pro Ser Ser Ala Gly Arg His Val Arg Ser Tyr Asn
            20                  25                  30
His Leu Gln Gly Asp Val Arg Trp Arg Lys Leu Phe Ser Phe Thr Lys
        35                  40                  45
Tyr Phe Leu Lys Ile Glu Lys Asn Gly Lys Val Ser Gly Thr Lys Lys
    50                  55                  60
Glu Asn Cys Pro Tyr Ser Ile Leu Glu Ile Thr Ser Val Glu Ile Gly
65                  70                  75                  80
Val Val Ala Val Lys Ala Ile Asn Ser Asn Tyr Tyr Leu Ala Met Asn
                85                  90                  95
Lys Lys Gly Lys Leu Tyr Gly Ser Lys Glu Phe Asn Asn Asp Cys Lys
            100                 105                 110
Phe Lys Glu Arg Ile Glu Glu Asn Gly Tyr Asn Thr Tyr Ala Ser Phe
        115                 120                 125
Asn Trp Gln His Asn Gly Arg Glu Met Tyr Val Ala Leu Asn Gly Lys
    130                 135                 140
Gly Ala Pro Arg Arg Gly Gln Lys Thr Arg Arg Lys Asn Thr Ser Ala
145                 150                 155                 160
His Phe Leu Pro Met Val Val His Ser
                165

<210> 9
<211> 169
<212> PRT
<213> Artificial Sequence

<220>
<223> Leu40-Ser208 fragment of SEQ ID NO: 1 + L152F+N181D

<400> 9
Leu Gly Gln Asp Met Val Ser Pro Glu Ala Thr Asn Ser Ser Ser Ser
1               5                   10                  15
Ser Phe Ser Ser Pro Ser Ser Ala Gly Arg His Val Arg Ser Tyr Asn
            20                  25                  30
His Leu Gln Gly Asp Val Arg Trp Arg Lys Leu Phe Ser Phe Thr Lys
        35                  40                  45
Tyr Phe Leu Lys Ile Glu Lys Asn Gly Lys Val Ser Gly Thr Lys Lys
    50                  55                  60
Glu Asn Cys Pro Tyr Ser Ile Leu Glu Ile Thr Ser Val Glu Ile Gly
65                  70                  75                  80
Val Val Ala Val Lys Ala Ile Asn Ser Asn Tyr Tyr Leu Ala Met Asn
                85                  90                  95
Lys Lys Gly Lys Leu Tyr Gly Ser Lys Glu Phe Asn Asn Asp Cys Lys
            100                 105                 110
Phe Lys Glu Arg Ile Glu Glu Asn Gly Tyr Asn Thr Tyr Ala Ser Phe
        115                 120                 125
Asn Trp Gln His Asn Gly Arg Gln Met Tyr Val Ala Leu Asp Gly Lys

130                 135                 140
Gly Ala Pro Arg Arg Gly Gln Lys Thr Arg Arg Lys Asn Thr Ser Ala
145                 150                 155                 160
His Phe Leu Pro Met Val Val His Ser
                165

<210> 10
<211> 169
<212> PRT
<213> Artificial Sequence

<220>
<223> Leu40-Ser208 fragment of SEQ ID NO: 1 + L152F+V123I+Q175E

<400> 10
Leu Gly Gln Asp Met Val Ser Pro Glu Ala Thr Asn Ser Ser Ser Ser
1               5                   10                  15
Ser Phe Ser Ser Pro Ser Ser Ala Gly Arg His Val Arg Ser Tyr Asn
            20                  25                  30
His Leu Gln Gly Asp Val Arg Trp Arg Lys Leu Phe Ser Phe Thr Lys
        35                  40                  45
Tyr Phe Leu Lys Ile Glu Lys Asn Gly Lys Val Ser Gly Thr Lys Lys
    50                  55                  60
Glu Asn Cys Pro Tyr Ser Ile Leu Glu Ile Thr Ser Val Glu Ile Gly
65                  70                  75                  80
Val Val Ala Ile Lys Ala Ile Asn Ser Asn Tyr Tyr Leu Ala Met Asn
                85                  90                  95
Lys Lys Gly Lys Leu Tyr Gly Ser Lys Glu Phe Asn Asn Asp Cys Lys
            100                 105                 110
Phe Lys Glu Arg Ile Glu Glu Asn Gly Tyr Asn Thr Tyr Ala Ser Phe
        115                 120                 125
Asn Trp Gln His Asn Gly Arg Glu Met Tyr Val Ala Leu Asn Gly Lys
    130                 135                 140
Gly Ala Pro Arg Arg Gly Gln Lys Thr Arg Arg Lys Asn Thr Ser Ala
145                 150                 155                 160
His Phe Leu Pro Met Val Val His Ser
                165

<210> 11
<211> 169
<212> PRT
<213> Artificial Sequence

<220>
<223> Leu40-Ser208 fragment of SEQ ID NO: 1 + L152F+V123I+N181D

<400> 11
Leu Gly Gln Asp Met Val Ser Pro Glu Ala Thr Asn Ser Ser Ser Ser
1               5                   10                  15
Ser Phe Ser Ser Pro Ser Ser Ala Gly Arg His Val Arg Ser Tyr Asn
            20                  25                  30
His Leu Gln Gly Asp Val Arg Trp Arg Lys Leu Phe Ser Phe Thr Lys
        35                  40                  45
Tyr Phe Leu Lys Ile Glu Lys Asn Gly Lys Val Ser Gly Thr Lys Lys
    50                  55                  60
Glu Asn Cys Pro Tyr Ser Ile Leu Glu Ile Thr Ser Val Glu Ile Gly
65                  70                  75                  80
Val Val Ala Ile Lys Ala Ile Asn Ser Asn Tyr Tyr Leu Ala Met Asn
                85                  90                  95
Lys Lys Gly Lys Leu Tyr Gly Ser Lys Glu Phe Asn Asn Asp Cys Lys
            100                 105                 110
Phe Lys Glu Arg Ile Glu Glu Asn Gly Tyr Asn Thr Tyr Ala Ser Phe
        115                 120                 125

Asn Trp Gln His Asn Gly Arg Gln Met Tyr Val Ala Leu Asp Gly Lys
    130                 135                 140
Gly Ala Pro Arg Arg Gly Gln Lys Thr Arg Arg Lys Asn Thr Ser Ala
145                 150                 155                 160
His Phe Leu Pro Met Val Val His Ser
                165

<210> 12
<211> 169
<212> PRT
<213> Artificial Sequence

<220>
<223> Leu40-Ser208 fragment of SEQ ID NO: 1 + L152F+Q175E+N181D

<400> 12
Leu Gly Gln Asp Met Val Ser Pro Glu Ala Thr Asn Ser Ser Ser Ser
1               5                   10                  15
Ser Phe Ser Ser Pro Ser Ser Ala Gly Arg His Val Arg Ser Tyr Asn
            20                  25                  30
His Leu Gln Gly Asp Val Arg Trp Arg Lys Leu Phe Ser Phe Thr Lys
        35                  40                  45
Tyr Phe Leu Lys Ile Glu Lys Asn Gly Lys Val Ser Gly Thr Lys Lys
    50                  55                  60
Glu Asn Cys Pro Tyr Ser Ile Leu Glu Ile Thr Ser Val Glu Ile Gly
65                  70                  75                  80
Val Val Ala Val Lys Ala Ile Asn Ser Asn Tyr Tyr Leu Ala Met Asn
                85                  90                  95
Lys Lys Gly Lys Leu Tyr Gly Ser Lys Glu Phe Asn Asn Asp Cys Lys
            100                 105                 110
Phe Lys Glu Arg Ile Glu Glu Asn Gly Tyr Asn Thr Tyr Ala Ser Phe
        115                 120                 125
Asn Trp Gln His Asn Gly Arg Glu Met Tyr Val Ala Leu Asp Gly Lys
    130                 135                 140
Gly Ala Pro Arg Arg Gly Gln Lys Thr Arg Arg Lys Asn Thr Ser Ala
145                 150                 155                 160
His Phe Leu Pro Met Val Val His Ser
                165

<210> 13
<211> 169
<212> PRT
<213> Artificial Sequence

<220>
<223> FGF10-STAB

<400> 13
Leu Gly Gln Asp Met Val Ser Pro Glu Ala Thr Asn Ser Ser Ser Ser
1               5                   10                  15
Ser Phe Ser Ser Pro Ser Ser Ala Gly Arg His Val Arg Ser Tyr Asn
            20                  25                  30
His Leu Gln Gly Asp Val Arg Trp Arg Lys Leu Phe Ser Phe Thr Lys
        35                  40                  45
Tyr Phe Leu Lys Ile Glu Lys Asn Gly Lys Val Ser Gly Thr Lys Lys
    50                  55                  60
Glu Asn Cys Pro Tyr Ser Ile Leu Glu Ile Thr Ser Val Glu Ile Gly
65                  70                  75                  80
Val Val Ala Ile Lys Ala Ile Asn Ser Asn Tyr Tyr Leu Ala Met Asn
                85                  90                  95
Lys Lys Gly Lys Leu Tyr Gly Ser Lys Glu Phe Asn Asn Asp Cys Lys
            100                 105                 110
Phe Lys Glu Arg Ile Glu Glu Asn Gly Tyr Asn Thr Tyr Ala Ser Phe

115                 120                 125
Asn Trp Gln His Asn Gly Arg Glu Met Tyr Val Ala Leu Asp Gly Lys
    130                 135                 140
Gly Ala Pro Arg Arg Gly Gln Lys Thr Arg Arg Lys Asn Thr Ser Ala
145                 150                 155                 160
His Phe Leu Pro Met Val Val His Ser
                165

<210> 14
<211> 169
<212> PRT
<213> Artificial Sequence

<220>
<223> Leu40-Ser208 fragment of SEQ ID NO: 1, FGF10-wt

<400> 14
Leu Gly Gln Asp Met Val Ser Pro Glu Ala Thr Asn Ser Ser Ser Ser
1               5                   10                  15
Ser Phe Ser Ser Pro Ser Ser Ala Gly Arg His Val Arg Ser Tyr Asn
            20                  25                  30
His Leu Gln Gly Asp Val Arg Trp Arg Lys Leu Phe Ser Phe Thr Lys
        35                  40                  45
Tyr Phe Leu Lys Ile Glu Lys Asn Gly Lys Val Ser Gly Thr Lys Lys
    50                  55                  60
Glu Asn Cys Pro Tyr Ser Ile Leu Glu Ile Thr Ser Val Glu Ile Gly
65                  70                  75                  80
Val Val Ala Val Lys Ala Ile Asn Ser Asn Tyr Tyr Leu Ala Met Asn
                85                  90                  95
Lys Lys Gly Lys Leu Tyr Gly Ser Lys Glu Phe Asn Asn Asp Cys Lys
            100                 105                 110
Leu Lys Glu Arg Ile Glu Glu Asn Gly Tyr Asn Thr Tyr Ala Ser Phe
        115                 120                 125
Asn Trp Gln His Asn Gly Arg Gln Met Tyr Val Ala Leu Asn Gly Lys
    130                 135                 140
Gly Ala Pro Arg Arg Gly Gln Lys Thr Arg Arg Lys Asn Thr Ser Ala
145                 150                 155                 160
His Phe Leu Pro Met Val Val His Ser
                165

<210> 15
<211> 190
<212> PRT
<213> Artificial Sequence

<220>
<223> Recombinant human FGF10-wt

<400> 15
Met Gly Ser Ser His His His His His His Ser Ser Gly Leu Val Pro
1               5                   10                  15
Arg Gly Ser His Met Leu Gly Gln Asp Met Val Ser Pro Glu Ala Thr
            20                  25                  30
Asn Ser Ser Ser Ser Ser Phe Ser Ser Pro Ser Ser Ala Gly Arg His
        35                  40                  45
Val Arg Ser Tyr Asn His Leu Gln Gly Asp Val Arg Trp Arg Lys Leu
    50                  55                  60
Phe Ser Phe Thr Lys Tyr Phe Leu Lys Ile Glu Lys Asn Gly Lys Val
65                  70                  75                  80
Ser Gly Thr Lys Lys Glu Asn Cys Pro Tyr Ser Ile Leu Glu Ile Thr
                85                  90                  95
Ser Val Glu Ile Gly Val Val Ala Val Lys Ala Ile Asn Ser Asn Tyr
            100                 105                 110

Tyr Leu Ala Met Asn Lys Lys Gly Lys Leu Tyr Gly Ser Lys Glu Phe
        115                 120                 125
Asn Asn Asp Cys Lys Leu Lys Glu Arg Ile Glu Glu Asn Gly Tyr Asn
    130                 135                 140
Thr Tyr Ala Ser Phe Asn Trp Gln His Asn Gly Arg Gln Met Tyr Val
145                 150                 155                 160
Ala Leu Asn Gly Lys Gly Ala Pro Arg Arg Gly Gln Lys Thr Arg Arg
                165                 170                 175
Lys Asn Thr Ser Ala His Phe Leu Pro Met Val Val His Ser
            180                 185                 190

<210> 16
<211> 136
<212> PRT
<213> Artificial Sequence

<220>
<223> FGF10 from Falco peregrinus having 88% sequence identity with SEQ
      ID NO: 3 and SEQ ID NO: 5

<400> 16
Leu Gln Gly Asp Val Arg Lys Arg Lys Leu Tyr Ser Tyr Asn Lys Tyr
1               5                   10                  15
Phe Leu Lys Ile Glu Lys Asn Gly Lys Val Ser Gly Thr Lys Lys Glu
            20                  25                  30
Asn Cys Pro Phe Ser Ile Leu Glu Ile Thr Ser Val Glu Ile Gly Val
        35                  40                  45
Val Ala Val Lys Ser Ile Lys Ser Asn Tyr Tyr Leu Ala Met Asn Lys
    50                  55                  60
Lys Gly Lys Val Tyr Gly Ser Lys Glu Phe Asn Ser Asp Cys Lys Leu
65                  70                  75                  80
Lys Glu Arg Ile Glu Glu Asn Gly Tyr Asn Thr Tyr Ala Ser Leu Asn
                85                  90                  95
Trp Lys His Asn Gly Arg Gln Met Phe Val Ala Leu Asn Gly Arg Gly
            100                 105                 110
Ala Thr Lys Arg Gly Gln Lys Thr Arg Arg Lys Asn Thr Ser Ala His
        115                 120                 125
Phe Leu Pro Met Val Val Met Ser
    130                 135

<210> 17
<211> 136
<212> PRT
<213> Artificial Sequence

<220>
<223> FGF10-Fp+L152F

<400> 17
Leu Gln Gly Asp Val Arg Lys Arg Lys Leu Tyr Ser Tyr Asn Lys Tyr
1               5                   10                  15
Phe Leu Lys Ile Glu Lys Asn Gly Lys Val Ser Gly Thr Lys Lys Glu
            20                  25                  30
Asn Cys Pro Phe Ser Ile Leu Glu Ile Thr Ser Val Glu Ile Gly Val
        35                  40                  45
Val Ala Val Lys Ser Ile Lys Ser Asn Tyr Tyr Leu Ala Met Asn Lys
    50                  55                  60
Lys Gly Lys Val Tyr Gly Ser Lys Glu Phe Asn Ser Asp Cys Lys Phe
65                  70                  75                  80
Lys Glu Arg Ile Glu Glu Asn Gly Tyr Asn Thr Tyr Ala Ser Leu Asn
                85                  90                  95
Trp Lys His Asn Gly Arg Gln Met Phe Val Ala Leu Asn Gly Arg Gly
            100                 105                 110

Ala Thr Lys Arg Gly Gln Lys Thr Arg Arg Lys Asn Thr Ser Ala His
        115                 120                 125
Phe Leu Pro Met Val Val Met Ser
    130                 135

<210> 18
<211> 584
<212> DNA
<213> Artificial Sequence

<220>
<223> Nucleotide sequence of recombinant human FGF10-wt

<400> 18
ccatgggcag cagccatcat catcatcatc acagcagcgg cctggtgccg cgcggcagcc      60

atatgctggg tcaagatatg gttagtccgg aagcaaccaa tagcagcagc agctcattta     120

gcagcccgag cagcgcaggt cgtcatgttc gtagctataa tcatctgcag ggtgatgttc     180

gttggcgtaa actgtttagc tttaccaaat acttcctgaa aatcgaaaag aacggtaaag     240

ttagcggcac caaaaaagaa aattgcccgt atagcattct ggaaattacc agcgttgaaa     300

ttggtgttgt tgccgtgaaa gcaatcaaca gcaactatta tctggccatg aacaaaaaag     360

gcaaactgta tggtagcaaa gagttcaaca acgactgcaa actgaaagaa cgcattgaag     420

aaaatggcta taatacctat gccagcttta actggcagca taatggtcgt cagatgtatg     480

ttgcactgaa tggtaaaggt gcaccgcgtc gtggtcagaa aacccgtcgt aaaaatacca     540

gcgcacattt tctgccgatg gttgttcata gctaatagct cgag                      584

## Description

### FIELD OF THE INVENTION

The present invention relates to thermostable engineered Fibroblast Growth Factor 10 (FGF10) or the fragment thereof having improved thermal stability compared to the wild-type FGF10 and the use thereof in cultivation of spheroids and organoids, regenerative medicine, or other related medical applications or cosmetics. The present invention further relates to a culture medium comprising FGF10 suitable for proliferation and differentiation of the human embryonic stem cells as well as formation and differentiation of spheroids, and organoids.

### BACKGROUND OF THE INVENTION

Fibroblast Growth Factor 10 (FGF10, also referred to as keratinocyte growth factor 2, KGF-2, KGF2 or rhKGF2) belongs to one of five paracrine-acting subfamilies of fibroblast growth factors that interact with heparin. Together with closely related FGF3, FGF7 and FGF22, FGF10 forms so-called FGF7 subfamily that is unique when compared to other FGFs because its members are secreted exclusively by the mesenchyme, and specifically activate the "b" isoforms of receptor FGFR1 (FGFR1b) and FGFR2 (FGFR2b) present in the overlying epithelium (Itoh, N. & Ornitz, D.M., 2004, Trends Genet. 20:563-569). FGF10 plays an important role in organogenesis and tissue patterning in the embryo, and mediates wound healing and tissue homeostasis in adult mammals via regulation of cell proliferation and cell differentiation (Beenken, A. & Mohammadi, M., 2009, Nat. Rev. Drug Discov. 8:235-253). FGF10 has been shown to mediate epithelial-mesenchymal interactions, which are essential to lung development and regeneration after various stresses (Tong, L. et al., 2016, Sci. Rep. 6:21642). FGF10 is further required for development of limb, thyroid, pituitary, kidney, teeth, hair follicles, salivary gland, inner ear, thymus, glandular stomach, and pancreas (Itoh, N., 2016, Cytokine Growth Factor Rev. 28:63-69).

The human FGF10 cDNA encodes a 208 amino acid residue protein with a hydrophobic N-terminal signal peptide of 37 amino acids. FGF10 is highly conserved among the vertebrate species, in both gene structure and amino-acid sequence (Ornitz, D.M & Itoh, N., 2001, Genome Biol. 2: 3005) Across the vertebrate species, orthologous FGF10 proteins share about 85% amino acid sequence identities as well as similar biological functions (Ornitz, D.M & Itoh, N., 2015, Rev. Dev. Biol. 4:215-266). FGF10 contains the conserved ~120-amino acid core region (amino acids 75-166 and 176-205) that is homologous (∼30-60%) to other paracrine FGF members (Emoto, H. et al., 1997, J. Biol. Chem. 272: 23191-23194) and that adopt a β-trefoil fold consisting of 12 β-strands (Yeh, B.K. et al., 2003, Proc. Natl. Acad. Sci. U. S. A. 100:2266-2271). The core region of FGF10 bears most of the amino acids that are important for interaction with FGFR2 receptor.

The selective interaction of FGF10 with FGFR2b receptor leads to autophosphorylation of the receptor and later to the activation of intracellular signalling pathways (RAS-MAPK, PI3K-AKT, and PLC*γ*1), that promote cell survival, induce a mitogenic cell response, and mediate cell mobility. Thanks to its ability to regulate epithelial proliferation, lineage commitment during embryonic development and post-natal life, and ability to stimulate epithelial regeneration after injury demonstrated on rodent animal models, the main therapeutical potential FGF10 lies in preventing alveolar cell injuries and restoring the normal epithelial function in damaged lungs (Yuan, T. et al., 2018, Front. Genet. 9:418). The other potential therapeutical applications of FGF10 includes wound healing (US 6,653,284 B2), treatment of heart diseases (Rochairs, F. et al., 2014, Cardiovasc. Res. 104:432-442), or treatment of obesity and metabolic diseases (Fisher, C. et al., 2017, Nat. Commun. 8:2079). Besides the therapeutic applications, FGF10 is often used as a component of cultivation media for various embryonic stem cells, spheroids and organoids (US 6,692,961 B1; Rabata, A. et al., 2020, Front. Cell Dev. Biol. 21:574).

Practical potential of FGF10 is, however, limited by its intrinsic instability demonstrated by its short half-life (lower than 24 hours in cell-free medium), low thermostability, and vulnerability to proteolytic degradation connected with rapid loss of its biological activity (Buchtova, M. et al., 2015, Cell. Mol. Life Sci., 72: 2445-2459; Chen, G. et al., 2012, Stem Cells 30:623-630). The low stability of FGF10 represents a significant obstacle for storage, transport, and practical use of this protein. Due to its low stability, FGF10 must be replaced frequently in cell cultures and the fluctuations of concentration may negatively influence cell fitness. The same goes for therapeutic applications as unfolded or aggregated protein forms arising as a result of the degradation may be toxic or immunogenic (Chen, B.L. & Arakawa, T., 1996, J. Pharm. Sci. 85:419-426).

A typical process of FGF10 stabilization is represented by addition of heparin. Heparin binding protects FGF10 from heat and acid denaturation and prolongs its half-life (Buchtova, M. et al., 2015, Cell. Mol. Life Sci., 72: 2445-2459). Nevertheless, heparin use is not physiological as it is produced exclusively by mast cells and its anticoagulation properties can further influence the fragile balance in the cellular environment. To use FGF10 in medical and cosmetic applications, different ways of obtaining higher stability must be developed.

Other strategies have been adopted to stabilize FGF10 without the use of heparin. The one described in WO 2013/082196 (or US 10,039,807) uses heparin mimicking sulfonate polymers and co-polymers in conjunction with FGF10 to achieve stability. Others use reducing agents (US 7,754,686), sucrose octa sulphate addition (US 5,202,311), hydroxypropyl cellulose addition (US 5,189,148), glucan sulphate addition (EP 0345660), polyanion-PEG conjugates (Khondee, S., et al., 2011, Biomacromolecules 12:3880-3894), or chelating agents (US 5,217,954) to stabilise FGF polypeptides. Patent document US 8,304,387 describes novel stable formulation of keratinocyte growth factor (KGF) useful for lyophilisation, where KGF is used in complex with sugars, surfactants, or bulking agents. EP 750628 B1 describes FGF10 produced by recombinant techniques. Such polypeptide, or polynucleotide encoding such polypeptide, can be used for therapeutic purposes, for example, promoting healing of wounds, burns and ulcers, to prevent neuronal damage due to neuronal disorders, and to prevent skin aging and hair loss. However, this form of polypeptide is not stabilized by any means.

An alternative approach to the addition of stabilizing additives is represented by protein engineering. Protein engineering introduces substitutions in the amino acid sequence that enhance the protein stability. Patent document EP 0950100 B1 relates to engineered forms of FGF10 polypeptide truncated at its N-terminal or C-terminal parts, and carrying the C-terminal substitutions R194E, R194Q, K191E, K191Q, R188E, R188Q that were constructed with a goal to enhance level of protein expression and solubility in *E. coli,* and that can be used in therapeutic applications. However, they neither state nor support that the mutations or the truncation would affect thermal stability or half-life of the presented FGF10 variants. Patent document US 6,653,284 B2 relates to liquid and lyophilized formulations of FGF10 polypeptides truncated either at the N-terminal or C-terminal parts for therapeutic use requiring soft issue growth and regeneration. For none of the truncated forms of FGF10, enhanced thermal stability or half-life have been demonstrated. Patent document WO 2001/002433 A1 relates to mutant forms of FGF10 polypeptides truncated at the N-terminal part and carrying the substitutions R68G, R68S, R68A, R78A, R80A, K81A, K87A, K91A, K136A, K137A, K139A, K144A, K148E, K149E, K151A, K153A, K155A, R174A, K183A, K183Q, K183E, R187A, R188A, R188E, or K191E that show enhanced activity, increased stability, higher yield, or better solubility and can be used in therapeutic application to promote wound healing. The other potentially stabilizing substitutions of FGF10 polypeptide, N105D, C106F, K144R, K153M, and I156R have been recently identified by *in silico* design, however their experimental verification have not been provided (Alizadeh, A.K., et al., 2021, Growth Factors 38:197-209).

### DISCLOSURE OF THE INVENTION

The present invention presents isolated thermostable engineered polypeptide that is derived from human FGF10 polypeptide (SEQ ID NO: 1) and can be used in cultivation of spheroids and organoids, regenerative medicine or other related medical applications, or cosmetics.





 (SEQ ID NO: 1)

The drawback resulting from natural instability of FGF10 polypeptide is overcome by the present invention that presents an isolated thermostable engineered polypeptide that possesses FGF10 activity and has 85% sequence identity to a sequence SEQ ID NO: 1 or a functional fragment thereof.

The subject-matter of the present invention is a thermostable FGF10 polypeptide possessing FGF10 activity and having or comprising at least 85% sequence identity of SEQ ID NO:3 that has an amino acid sequence from Ser69 to Ser208 of SEQ ID NO:1, or a fragment thereof, wherein the thermostable FGF10 polypeptide or the fragment thereof comprising at least an amino acid substitution L152F.

According to a prefered embodiment of the present invention the thermostable FGF10 polypeptide has or comprises SEQ ID NO:3, or the fragment thereof, wherein a thermostable FGF10 polypeptide or the fragment thereof comprising at least an amino acid substitution L152F.

Prefered embodiment of the present invention discloses the thermostable FGF10 polypeptide or the fragment thereof further comprising at least one, or at least two, or at least three amino acid substitutions selected from a group consisting of V123I, Q175E, and N181D. This means that the polypeptide according to the invention always exhibits at least L152F substitution; or combination of two, three or four substitutions combining L152F with V123I and/or Q175E and/or N181D.

The amino acid sequence of human FGF10 polypeptide (SEQ ID NO: 1) consists of 208 amino acids, where N-terminal amino acid region MWKWILTHCA SAFPHLPGCC CCCFLLLFLV SSVPVTC (SEQ ID NO: 2) of the amino acid sequence SEQ ID NO: 1 corresponds to an N-terminal signal peptide, that directs the protein translocation and that is usually removed in the mature polypeptide. Signal peptide thus may or may not be present in the amino acid sequence of FGF10 polypeptide according to the present invention. In some embodiments, such as recombinant production of FGF10 polypeptide in *E. coli,* it may be replaced just by single amino acid methionine (M).

The mature human FGF10 polypeptide may be further truncated at its N-terminus up to 31 amino acids without loss of its biological activity and thus consisting of amino acid sequence Ser69-Ser208 with or without N-terminal Met (amino acid numbering corresponds to SEQ ID NO: 1).

It is further known in the art that FGF10 polypeptides of various vertebrate species have about 85% sequence identity, while performing analogous functions and having similar biological activity.

The subject of the present invention relates to an isolated engineered thermostable FGF10 polypeptide exhibiting FGF10 activity and comprising:
(a) The thermostable FGF10 polypeptide, wherein the said polypeptide has or comprises at least 85% sequence identity to an amino acid sequence from Ser69 to Ser208 of SEQ ID NO: 1 (see SEQ ID NO: 3);
(b) the thermostable FGF10 polypeptide having or comprising SEQ ID NO: 3 or the fragment thereof; and
(c) the thermostable polypeptide of (a or b), wherein said polypeptide has at least stabilizing amino acid substitution L152F (SEQ ID NO: 3), or the combination of stabilizing substitution L152F with at least one additional stabilizing amino acid substitutions selected from the group consisting of V123I, Q175E, or N181D.

SY NHLQGDVRWR KLFSFTKYFL KIEKNGKVSG TKKENCPYSI
LEITSVEIGV VAVKAINSNY YLAMNKKGKL YGSKEFNNDC KFKERIEENG
YNTYASFNWQ HNGRQMYVAL NGKGAPRRGQ KTRRKNTSAH FLPMVVHS (SEQ ID
NO: 3)

Preferably, the length of the thermostable FGF10 polypeptide sequence is up to 228 amino acids, more preferably up to 190 amino acids.

The amino acid marked in bold and underlined in SEQ ID NO: 3 is mutated in the position 84 of this amino acid sequence; which corresponds to the amino acid substitution L152F in the amino acid numbering according to SEQ ID NO: 1.

In some embodiments, the thermostable polypeptide further comprises at least one, or at least two, or at least three of the following amino acid substitutions:
- amino acid substitution I in the position 55 of the said amino acid sequence SEQ ID NO: 3 (corresponding to the amino acid substitution V123I in the numbering according to SEQ ID NO: 1);
- amino acid substitution E in the position 107 of the said amino acid sequence SEQ ID NO: 3 (corresponding to the amino acid substitution Q175E in the numbering according to SEQ ID NO: 1);
- amino acid substitution D in the position 113 of the said amino acid sequence SEQ ID NO: 3 (corresponding to the amino acid substitution N181D in the numbering according to SEQ ID NO: 1).

In some embodiments, the thermostable FGF10 polypeptide may carry at the N-terminal part of SEQ ID NO: 3 at least part or all of the amino acid sequence MWKWILTHCA SAFPHLPGCC CCCFLLLFLV SSVPVTCQAL GQDMVSPEAT NSSSSSFSSP SSAGRHVR (SEQ ID NO: 4). In other embodiments, the thermostable FGF10 polypeptide may carry at the N-terminal part of SEQ ID NO: 3 methionine (M). In some embodiments, the thermostable FGF10 polypeptide may carry at its N-terminal or C-terminal parts short fusion amino acid sequences (known as tags) up to a maximum length of 20 amino acids. Said embodiments of the extensions at the N-terminus and C-terminus of the thermostable FGF10 polypeptide sequence can be combined.

According to the other prefered embodiment of the present invention the thermostable FGF10 polypeptide further carries:
- at least part or all amino acids of SEQ ID NO: 4 or methionine at the N-terminus of SEQ ID NO: 3;
   and/or
- at least one short fusion amino acid sequence (tag) up to a maximum length of 20 amino acids at the N-terminal or C-terminal parts.

In a preferred embodiment, the present invention discloses a thermostable FGF10 polypeptide having or comprising a sequence with at least 85% sequence identity to the sequence SEQ ID NO: 5:

L GQDMVSPEAT NSSSSSFSSP SSAGRHVRSY NHLQGDVRWR
KLFSFTKYFL KIEKNGKVSG TKKENCPYSI LEITSVEIGV VAVKAINSNY
YLAMNKKGKL YGSKEFNNDC KFKERIEENG YNTYASFNWQ HNGRQMYVAL
NGKGAPRRGQ KTRRKNTSAH FLPMVVHS (SEQ ID NO: 5)

Wherein the amino acid marked in bold and underlined must be retained.

Preferably, the length of the thermostable FGF10 polypeptide sequence is up to 228 amino acids, more preferably up to 190 amino acids.

The amino acid sequence SEQ ID NO: 5 corresponds to the Leu40 - Ser208 fragment of SEQ ID NO: 1. The amino acid marked in bold and underlined in SEQ ID NO: 5 is mutated in the position 113 of this amino acid sequence; which corresponds to the amino acid substitution L152F in the numbering according to SEQ ID NO: 1.

According to the other prefered embodiment of the present invention the thermostable FGF10 polypeptide has or comprises at least 85% sequence identity of SEQ ID NO:5 that has an amino acid sequence from Leu40 to Ser208 of SEQ ID NO:1, or the fragment thereof, wherein the thermostable FGF10 polypeptide or the fragment thereof comprises at least an amino acid substitution L152F.

According to the other prefered embodiment of the present invention the thermostable FGF10 polypeptide has or comprises SEQ ID NO:5, or the fragment thereof, wherein a thermostable FGF10 polypeptide or the fragment thereof comprises at least an amino acid substitution L152F.

In some embodiments, the thermostable FGF10 polypeptide further comprises at least one, or at least two, or at least three of the following amino acid substitutions:
- amino acid substitution I in the position 84 of the said amino acid sequence SEQ ID NO: 5 (corresponding to the amino acid substitution V123I in the numbering according to SEQ ID NO: 1);
- amino acid substitution E in the position 136 of the said amino acid sequence SEQ ID NO: 5 (corresponding to the amino acid substitution Q175E in the numbering according to SEQ ID NO: 1);
- amino acid substitution D in the position 142 of the said amino acid sequence SEQ ID NO: 5 (corresponding to the amino acid substitution N181D in the numbering according to SEQ ID NO: 1).

According to the other prefered embodiment of the present invention the thermostable FGF10 polypeptide or the fragment thereof further comprises at least one, or at least two, or at least three amino acid substitution selected from a group consisting of V123I, Q175E, and N181D.

In some embodiments, the thermostable polypeptide may carry at the N-terminal part of SEQ ID NO. 5 at least part or all of the amino acid sequence MWKWILTHCA SAFPHLPGCC CCCFLLLFLV SSVPVTCQA (SEQ ID NO: 6). In other embodiments, the thermostable polypeptide may carry at the N-terminal part of SEQ ID NO: 5 methionine (M). In some embodiments, the thermostable polypeptide may carry at its N-terminal or C-terminal parts short fusion amino acid sequences (known as tags) up to a maximum length of 20 amino acids. Said embodiments of the extensions at the N-terminus and C-terminus of the thermostable polypeptide sequence can be combined.

In the detailed description of the invention and in the examples of the invention, the numbering of amino acid substitutions according to the sequence SEQ ID NO: 1 is used, i.e., numbering relative to the entire amino acid sequence of human FGF10 polypeptide, even when truncated amino acid sequences are used.

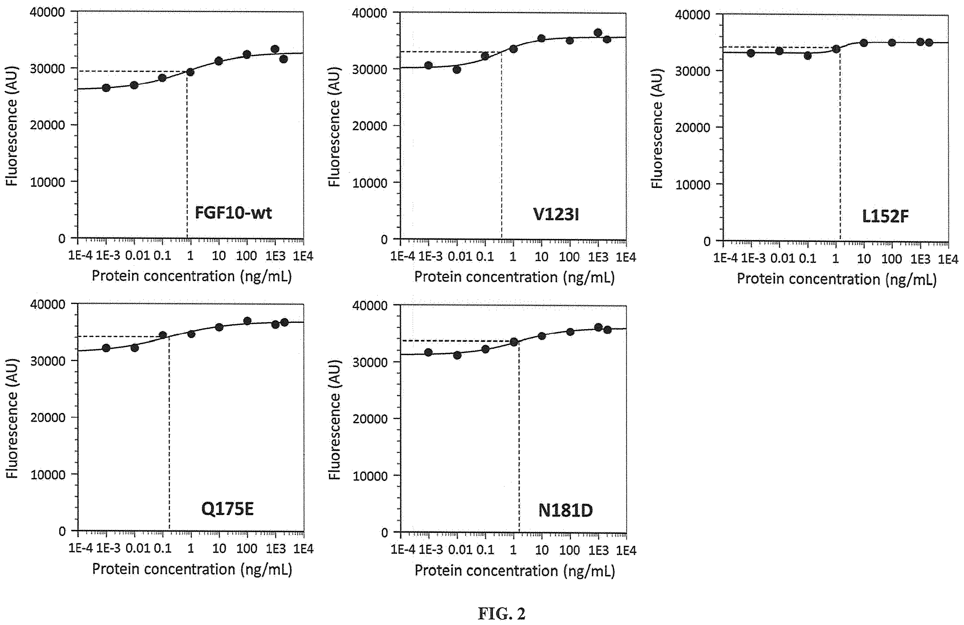

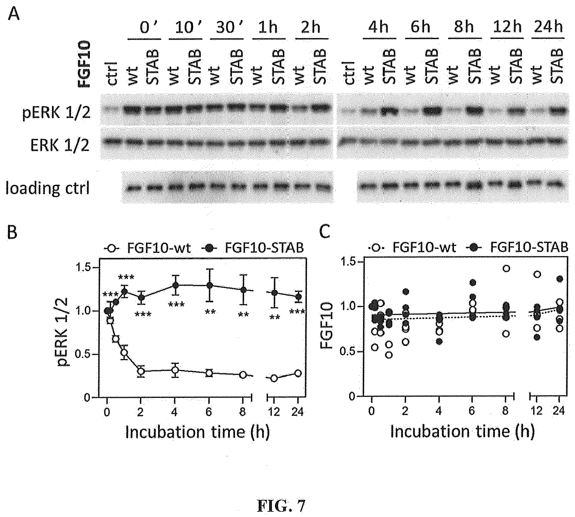

The thermostable FGF10 polypeptides or the fragments thereof according to the present invention possess unmodified FGF10 activity in 4MBr-5 cell proliferation assay, where lung epithelial 4MBr-5 cells express the FGF receptor 2b (FGFR2b), which is a natural ligand of the FGF10 polypeptide (see Figures 2, 6, 14, and 17). Advantageously subjected thermostable FGF10 polypeptides or the fragments thereof according to the invention show stable and unchanged biological activity at 37°C for prolonged periods of time (see Figures 7, 9, and 10).

The thermostable FGF10 polypeptides of the present invention are particularly advantageous because they are significantly more stable when compared with FGF10 wild-type or the fragment thereof. This stability is due to the inherent stability of the polypeptide molecule. No other compounds promoting protein stability, such as additives (i.e., heparin) or carrier molecules (i.e., BSA), have to be added. At the same time, the biological activity of thermostable FGF10 polypeptides is preserved. Disclosed thermostable FGF10 polypeptides can be used in research and clinical applications.

The thermostable FGF10 polypeptides or the fragments thereof according to the present invention possess unmodified FGF10 activity and increased melting temperature by 7 to 19°C, preferably by 10 to 19°C compared to the wild-type FGF10 polypeptide. All thermostable FGF10 polypeptides according to the present invention were constructed by side-directed mutagenesis or using the artificial gene synthesis, subcloned into expression vector pET28b, purified to homogeneity (protein purity ≥ 95% as determined by SDS-PAGE electrophoresis) and subsequently characterized for stability (melting temperature determination) and biological activity.

More preferred are thermostable FGF10 polypeptides up to 228 amino acids in total length (more preferably up to 190 amino acids in total length) having or comprising at least 85% sequence identity to the amino acid sequences selected from SEQ ID NO: 5, and SEQ ID NO: 7 to SEQ ID NO: 13 or the fragments thereof.

L GQDMVSPEAT NSSSSSFSSP SSAGRHVRSY NHLQGDVRWR
KLFSFTKYFL KIEKNGKVSG TKKENCPYSI LEITSVEIGV VAVKAINSNY
YLAMNKKGKL YGSKEFNNDC KFKERIEENG YNTYASFNWQ HNGRQMYVAL
NGKGAPRRGQ KTRRKNTSAH FLPMVVHS (SEQ ID NO: 5)

L GQDMVSPEAT NSSSSSFSSP SSAGRHVRSY NHLQGDVRWR
KLFSFTKYFL KIEKNGKVSG TKKENCPYSI LEITSVEIGV VAIKAINSNY
YLAMNKKGKL YGSKEFNNDC KFKERIEENG YNTYASFNWQ HNGRQMYVAL
NGKGAPRRGQ KTRRKNTSAH FLPMVVHS (SEQ ID NO: 7)

L GQDMVSPEAT NSSSSSFSSP SSAGRHVRSY NHLQGDVRWR
KLFSFTKYFL KIEKNGKVSG TKKENCPYSI LEITSVEIGV VAVKAINSNY
YLAMNKKGKL YGSKEFNNDC KFKERIEENG YNTYASFNWQ HNGREMYVAL
NGKGAPRRGQ KTRRKNTSAH FLPMVVHS (SEQ ID NO: 8)

L GQDMVSPEAT NSSSSSFSSP SSAGRHVRSY NHLQGDVRWR
KLFSFTKYFL KIEKNGKVSG TKKENCPYSI LEITSVEIGV VAVKAINSNY
YLAMNKKGKL YGSKEFNNDC KFKERIEENG YNTYASFNWQ HNGRQMYVAL
DGKGAPRRGQ KTRRKNTSAH FLPMVVHS (SEQ ID NO: 9)

L GQDMVSPEAT NSSSSSFSSP SSAGRHVRSY NHLQGDVRWR
KLFSFTKYFL KIEKNGKVSG TKKENCPYSI LEITSVEIGV VAIKAINSNY
YLAMNKKGKL YGSKEFNNDC KFKERIEENG YNTYASFNWQ HNGREMYVAL
NGKGAPRRGQ KTRRKNTSAH FLPMVVHS (SEQ ID NO: 10)

L GQDMVSPEAT NSSSSSFSSP SSAGRHVRSY NHLQGDVRWR
KLFSFTKYFL KIEKNGKVSG TKKENCPYSI LEITSVEIGV VAIKAINSNY
YLAMNKKGKL YGSKEFNNDC KFKERIEENG YNTYASFNWQ HNGRQMYVAL
DGKGAPRRGQ KTRRKNTSAH FLPMVVHS (SEQ ID NO: 11)

L GQDMVSPEAT NSSSSSFSSP SSAGRHVRSY NHLQGDVRWR
KLFSFTKYFL KIEKNGKVSG TKKENCPYSI LEITSVEIGV VAVKAINSNY
YLAMNKKGKL YGSKEFNNDC KFKERIEENG YNTYASFNWQ HNGREMYVAL
DGKGAPRRGQ KTRRKNTSAH FLPMVVHS (SEQ ID NO: 12)

L GQDMVSPEAT NSSSSSFSSP SSAGRHVRSY NHLQGDVRWR
KLFSFTKYFL KIEKNGKVSG TKKENCPYSI LEITSVEIGV VAIKAINSNY
YLAMNKKGKL YGSKEFNNDC KFKERIEENG YNTYASFNWQ HNGREMYVAL
DGKGAPRRGQ KTRRKNTSAH FLPMVVHS (SEQ ID NO: 13)

According to the other preferred embodiment of the present invention the thermostable FGF10 polypeptide is selected from a group of the thermostable FGF10 polypeptides comprising SEQ ID NO:5, SEQ ID NO:7, SEQ ID NO:8, SEQ ID NO:9, SEQ ID NO:10, SEQ ID NO:11, SEQ ID NO:12, or SEQ ID NO:13.

In some embodiments, the thermostable polypeptide of any of the sequences SEQ ID NO: 5, and SEQ ID NO: 7 to SEQ ID NO: 13 may carry at its N-terminal part at least part or all of the amino acid sequence MWKWILTHCA SAFPHLPGCC CCCFLLLFLV SSVPVTCQA (SEQ ID NO: 6). In other embodiments, the thermostable polypeptide of any of the sequences SEQ ID NO: 5, and SEQ ID NO: 7 to SEQ ID NO: 13 may carry at its N-terminal part methionine (M). In some embodiments, the thermostable polypeptide of any of the sequences SEQ ID NO: 5, and SEQ ID NO: 7 to SEQ ID NO: 13 may carry at its N-terminal or C-terminal parts short fusion amino acid sequences (known as tags) up to a maximum length of 20 amino acids. Said embodiments of the extensions at the N-terminus and C-terminus of the thermostable polypeptide sequence can be combined.

According to the other prefered embodiment of the present invention the thermostable FGF10 polypeptide further carries:
- at least part or all amino acids of SEQ ID NO:6 or methionine at the N-terminus of SEQ ID NO:5;
   and/or
- at least one short fusion amino acid sequence (tag) up to a maximum length of 20 amino acids at the N-terminal or C-terminal parts.

Another embodiments of the present invention disclose a thermostable FGF10 polypeptide possesing FGF10 activity wherein the thermostable FGF10 polypeptide according the present invention further carries SEQ ID NO:6 at the N-terminus any of SEQ ID NO:5, SEQ ID NO:7, SEQ ID NO:8, SEQ ID NO:9, SEQ ID NO:10, SEQ ID NO:11, SEQ ID NO:12, or SEQ ID NO:13.

The biological activity of FGF10 polypeptides, or fragments thereof, or muteins thereof according to the present invention can be quantitatively expressed by median effective dose, ED₅₀ (concentration of polypeptide needed to achieve half of the maximal biological effect), for the proliferation of 4MBr-5 cells, with an EC₅₀ for this effect < 15 ng/mL, preferably < 12 ng/mL. The biological activity of FGF10 can be evaluated by a cultured lung epithelial proliferation assay as previously described (Rubin, J.S. et al., 1989, Proc Natl Acad Sci U S A, 86:802-806).

More preferred embodiments of the present invention disclose the thermostable FGF10 polypeptide according to the present invention for use in regenerative medicine, preferably for lung epithelial regeneration after various stresses or for curing of wounds and ulcers or other related medical applications, such as cardiac treatment.

More preferred embodiments of the present invention further disclose the use the thermostable FGF10 polypeptide according to the present invention in cosmetics, preferably support of collagen synthesis, skin strength, anti-aging treatment, and non-therapeutic stimulation of hair growth.

More preferred embodiments of the present invention disclose a culture medium for proliferation and differentiation of the human embryonic stem cells, or formation and differentiation of spheroids and organoids, preferably lung organoids, stomach organoids, liver organoids, pancreas organoids, and prostate organoids comprising an effective amount of the thermostable FGF10 polypeptide according to the present invention in the range of 10 ng/mL to 500 ng/mL of culture medium.

These and other features, objects, and advantages of the present invention will become better understood from the description that follows. In the description, reference is made to the accompanying drawings, which form a part hereof and in which there is shown by way of illustration, not limitation, embodiments of the invention.

### DETAILED DESCRIPTION OF THE INVENTION

### DEFINITIONS

The definition of certain terms as used in this specification are provided below. Unless otherwise defined, all technical and scientific terms used herein have the same meaning as commonly understood by one of ordinary skill in the art to which this invention pertains.

The standard one-letter abbreviation for amino acids is used in the present invention, to describe the amino acid sequences of FGF10 polypeptides:
Alanine: A, cysteine: C, aspartic acid: D, glutamic acid: E, phenylalanine: F, glycine: G, histidine: H, isoleucine: L, lysine: K, leucine: L, methionine: M, asparagine: N, proline: P, glutamine: Q, arginine: R, serine: S, threonine: T, valine: V, tryptophane: W, and tyrosine: Y.

As used herein, the term 'thermostability' is synonymous with the term 'thermal stability' of the protein and encompasses thermodynamic and kinetic stabilities. Thermodynamic stability is related to the equilibrium between folded (native, functional protein) and unfolded or partially-unfolded state of the protein and is defined as the difference in Gibbs free energy between these two protein states. Kinetic stability reflects the rate (i.e., kinetics) of protein folding and unfolding and is related to a free-energy barrier 'separating' the native state of the protein from the non-functional forms (unfolded or partially unfolded states, irreversibly-denatured protein, aggregated protein).

As used herein, the term 'melting temperature' (*T*ₘ) of FGF10 protein refers to the temperature at which 50% of the protein is folded and 50% of the protein is unfolded. The melting temperature is a direct measure of thermodynamic stability. *T*ₘ can be determined by any suitable method known in the art such as circular dichroism (CD) spectroscopy, differential scanning calorimetry (DSC) or differential scanning fluorimetry (DSF) as further discussed in the Examples below. CD spectroscopy is a method suitable for monitoring the secondary structure and conformational changes of proteins as a function of temperature or other variable physico-chemical parameter (such as concentration of salts, denaturants, or organic cosolvents). DSC is a thermodynamical method that allows direct assessment of the heat energy uptake, which occurs in a protein sample within a regulated increase or decrease in temperature when the structural changes occur. DSF is a method that monitors changes in the overall tertiary structure of proteins as a function of temperature via following the changes in the intrinsic fluorescence.

As used herein, the term 'structural half-life' or 'half-life of the secondary structure' of FGF10 polypeptide refers to the amount of time it takes for the ellipticity, as a measure of protein secondary structure, to become reduced by half under defined process conditions. The half-life is a direct measure of the kinetic stability.

As used herein, the term 'wild-type' refers to native FGF10 having the most common amino acid sequence among members of species. Herein, wild-type FGF10 is the recombinant mature form of human FGF10 corresponding to the Leu40 - Ser208 fragment of SEQ ID NO: 1 which is 19.1 kDa protein consisting of 169 amino acids (SEQ ID NO: 14):

L GQDMVSPEAT NSSSSSFSSP SSAGRHVRSY NHLQGDVRWR
KLFSFTKYFL KIEKNGKVSG TKKENCPYSI LEITSVEIGV VAVKAINSNY
YLAMNKKGKL YGSKEFNNDC KLKERIEENG YNTYASFNWQ HNGRQMYVAL
NGKGAPRRGQ KTRRKNTSAH FLPMVVHS (SEQ ID NO: 14)

Preferably, the 'wild-type' FGF10 refers to the recombinant mature form of human FGF10 that contains a His-tag and a thrombin cleavage site at its N-terminal part with a molecular weight of 21.4 kDa and a length of 190 amino acids (SEQ ID NO: 15):

MGSSHHHHHH SSGLVPRGSH MLGQDMVSPE ATNSSSSSFS SPSSAGRHVR
SYNHLQGDVR WRKLFSFTKY FLKIEKNGKV SGTKKENCPY SILEITSVEI
GVVAVKAINS NYYLAMNKKG KLYGSKEFNN DCKLKERIEE NGYNTYASFN
WQHNGRQMYV ALNGKGAPRR GQKTRRKNTS AHFLPMVVHS (SEQ ID NO: 15)

Short fusion amino acids sequences, so-called tags or peptide tags, with a maximum length of up to 20 amino acids facilitate the expression, solubility, purification, and detection of the recombinant polypeptides. A person skilled in the art will know that, in general, the attaching of the tag into the N-terminal or C-terminal part of the polypeptide does not significantly affect its biological function (such as biological activity or stability). The tags, their amino acid sequences and their usage are known to those skilled in the art. The commonly used tags include, e.g., ALFA-tag, AviTag, C-tag, polyglutamate tag, polyarginine tag, E-tag, FLAG-tag, HA-tag, His-tag, Myc-tag, NE-tag, Rho1D4-tag, S-tag, Softag1, Softag3, Spot-tag, Strep-tag, T7-tag, TC tag, Ty tag, V5 tag, VSV-tag, Xpress tag, Isopeptag, SpyTag, SnoopTag, DogTag, or SdyTag.

As used herein, the term 'polypeptide derived from human FGF10' or 'thermostable FGF10 polypeptide' refers to polypeptide possessing FGF10 activity and having or comprising at least 85% sequence identity with SEQ ID NO: 3 or SEQ ID NO: 5.

As used herein, the term 'two-point mutant of FGF10' refers to the thermostable FGF10 polypeptide having the SEQ ID NO: 5 or the fragment thereof, and comprising the amino acid substitution L152F in combination with one additional amino acid substitution selected from the group consisting of: V123I, Q175E, and N181D. Preferably, it is the thermostable FGF10 polypeptide having the sequence SEQ ID NO: 7 (comprising the substitutions L152F and V123I), the thermostable FGF10 polypeptide having the sequence SEQ ID NO: 8 (comprising the substitutions L152F and Q175E), or the thermostable FGF10 polypeptide having the sequence SEQ ID NO: 9 (comprising the substitutions L152F and N181D).

As used herein, the term 'multiple-point mutant of FGF10' refers to the thermostable FGF10 polypeptide having the SEQ ID NO: 5 or the fragment thereof, and comprising the amino acid substitution L152F in combination with at least two or the three additional amino acid substitutions selected from the group consisting of: V123I, Q175E, and N181D. Preferably, it is the thermostable FGF10 polypeptide having the sequence SEQ ID NO: 10 (comprising the substitutions L152F, V123I, and Q175E), the thermostable FGF10 polypeptide having the sequence SEQ ID NO: 11 (comprising the substitutions L152F, V123I, and N181D), the thermostable FGF10 polypeptide having the sequence SEQ ID NO: 12 (comprising the substitutions L152F, Q175E, and N181D), or the thermostable FGF10 polypeptide having the sequence SEQ ID NO: 13 (comprising the substitutions L152F, V123I, Q175E, and N181D).

As used herein, the term '4-point mutant of FGF10' is synonymous with 'FGF10-STAB' and refers to the thermostable FGF10 polypeptide having the sequence SEQ ID NO: 5 or the fragment thereof, comprising the amino acid substitutions L152F, V123I, Q175E, and N181D. Preferably, it is the thermostable FGF10 polypeptide having the sequence SEQ ID NO: 13.

As used herein, the term 'FGF10 from *Falco peregrinus'* is synonymous with 'FGF10-Fp' and refers to FGF10 polypeptide having 88% sequence identity to SEQ ID NO: 3 or SEQ ID NO: 5 corresponding to the functional fragments of FGF10 polypeptide carrying the amino acid sequences Ser69-Ser208 or Leu40- Ser208, respectively, of SEQ ID NO: 1. FGF10-Fp polypeptide carrying modification in 16 of 136 amino acids, when compared with both SEQ ID NO: 3 or SEQ ID NO: 5, possesses unmodified FGF10 activity (as set forth in Example 14), and has amino acid sequence SEQ ID NO: 16.

LQGDVRKRK LYSYNKYFLK IEKNGKVSGT KKENCPFSIL EITSVEIGVV
AVKSIKSNYY LAMNKKGKVY GSKEFNSDCK LKERIEENGY NTYASLNWKH
NGRQMFVALN GRGATKRGQK TRRKNTSAHF LPMVVMS (SEQ ID NO: 16)

As used herein, the term 'single point variant of FGF10-Fp' is synonymous with 'FGF10-Fp+L1 52F' and refers to the thermostable FGF10 polypeptide comprising the stabilizing amino acid substitution F in the position 80 of the amino acid sequence SEQ ID NO: 16 corresponding to the substitution L152F in the numbering according to SEQ ID NO: 1 and having 88% sequence identity with SEQ ID NO: 3 or SEQ ID NO: 5. FGF10-Fp+L152F exhibits enhanced stability when compared with SEQ ID NO: 16, unmodified FGF10 activity (as set forth in Example 14) and has amino acid sequence SEQ ID NO: 17.

LQGDVRKRK LYSYNKYFLK IEKNGKVSGT KKENCPFSIL EITSVEIGVV
AVKSIKSNYY LAMNKKGKVY GSKEFNSDCK FKERIEENGY NTYASLNWKH
NGRQMFVALN GRGATKRGQK TRRKNTSAHF LPMVVMS (SEQ ID NO: 17)

As used herein, the term 'polypeptide derived from human FGF10' is synonymous with FGF10 mutant' and 'FGF10 mutein' and refers to a modified FGF10 polypeptide sequence that has at least one different amino acid sequence exhibiting any of the substitutions according to the invention as compared to human FGF10 polypeptide (SEQ ID NO: 1) or a fragment thereof.

As used herein, the term 'polypeptide' is synonymous with 'protein'. The polypeptides of the present invention may be recombinant polypeptides, natural polypeptides, or synthetic polypeptides, preferably recombinant polypeptides.

As used herein, the term 'activity of FGF10' is synonymous with 'biological activity of FGF10'. It intends the biological activity of FGF10 polypeptides or polypeptides derived from human FGF10 polypeptide, or fragments thereof, or muteins thereof according to the invention. The active (functional) polypeptide is represented by an intact portion of the amino acid sequence and structure of the subjected FGF10 polypeptide according to the invention, wherein the FGF10 polypeptide comprises all functionally important amino acids that are responsible for binding iterations with the cell surface receptors, heparan sulphates or their structural analogue heparin. Amino acids of FGF10 that are essential for the polypeptide function (such as those critical for the polypeptide-receptor interaction) can be identified by methods well known in the art, such as structural analysis including X-ray crystallography or nuclear magnetic resonance and site-directed mutagenesis or alanine-scanning mutagenesis. The functionally important amino acids of FGF10 polypeptide can be found in Yeh, K.B. et al., 2003, Proc Natl Acad Sci U S A, 100:2266-2271; and Zinkle, A. & Mohammadi, M., 2019, Front. Genet. 10:102.

Biological function of FGF10 polypeptide in tissue development and repair is driven through binding to fibroblast growth factor receptor 2 (FGFR2). Of the FGFR isoforms "b" (IIIb) and "c" (IIIc) that are generated by alternative splicing of the extracellular immunoglobulin domain III, FGF10 acts mostly via its selective interaction with FGFR2b receptor. Binding interaction of FGF10 with FGFR2b results in dimerization and autophosphorylation of the receptor and later to the activation of intracellular signalling pathways (RAS-MAPK, PI3K-AKT, and PLC*γ*1), which promotes cell survival, induces a mitogenic cell response, and mediates cell mobility. FGF10 polypeptide is predominantly expressed in the lung mesenchyme where it plays essential role in the regulation of epithelial proliferation and lineage commitment during embryonic lung development and post-natal life. Other target cells include cells of epithelial origin in general, such as cells involved in development of limbs, in both small and large intestine and various other organs including kidneys, thymus, liver, and heart, known in the art to express FGFR2b receptor or to respond to FGF10.

Biological activity can be measured by methods known in the art, for example as cell proliferation and/or substrate phosphorylation. Thus, 'a polypeptide having FGF10 activity' includes polypeptides that exhibit the FGF10 activity, in the cell proliferation assay (such as lung epithelial 4MBr-5 cell proliferation assay set forth in Examples 4, 8, 13, and 14), and in substrate phosphorylation (such as ERK1/2 phosphorylation set forth in Examples 9, and 10), and bind to the FGFR2b receptor.

The thermostable FGF10 polypeptide, or fragment thereof, or mutein thereof according to the invention is considered to have FGF10 activity if it exhibits the ED₅₀ value (median effective dose) determined by 4MBr-5 cell proliferation assay ≤ 15 ng/mL, preferably < 12 ng/mL. Although the degree of activity need not be identical to that of the FGF10 protein, preferably, 'a polypeptide having FGF10 activity' will exhibit substantially similar activity as compared to the FGF10-wt (i.e., the candidate polypeptide will exhibit greater activity or not more than about twofold less activity relative to the reference FGF10 protein).

As used herein, the term 'median effective dose' is synonymous with 'ED₅₀ value' and refers to the concentration of thermostable FGF10 polypeptide, or fragment thereof, or mutein thereof according to the invention needed to achieve half of the maximal proliferation (i.e., to achieve 50% of the maximum biological activity).

Biological activity of FGF10 polypeptide requires the presence of functional fragment of thermostable FGF10 polypeptide that retains the biological activity of FGF10. As used herein, the term 'fragment' is synonymous with 'functional fragment' and refers to the intact portion of the thermostable FGF10 polypeptide sequence and structure bearing all functionally important amino acids, i.e., amino acids responsible for the interaction of the polypeptide with FGF receptors and the interaction with heparan sulphates. It is known to those skilled in the art that FGF10 activity remains preserved after truncation of 37 (corresponding to the mature FGF10 polypeptide lacking the signal sequence) to 68 amino acids at the N-terminal part of FGF10 sequence (SEQ ID NO: 1). In addition, the mimetic peptides of FGF10, the shorter FGF10 peptides mimicking the FGF10 activity by binding to, and activating, or inhibiting the FGFR2b receptor have been previously described (see patent documents US 6,653,284 B2 and EP 0950100 A1).

FGF10 polypeptides among various vertebrate species have about 85% sequence identity. Therefore, a person skilled in the art will know that 85% of sequence identity does not negatively affect the biological activity of FGF10 polypeptide. The preferred is at least 90%, 91%, 92%, 93%, 94%, 95%, 96%, 97%, 98%, 99%, and 100% sequence identity. The thermostable FGF10 polypeptide also exhibits at least one or more stabilizing amino acid substitutions according to the invention. Thus, the subjected thermostable FGF10 polypeptides according to the invention have an increased *T*ₘ by at least 7°C, preferably by at least 10°C, more preferably by at least 14°C and simultaneously, retain the desired biological activity, i.e., are able to bind target FGF receptor on the surface of the cells to trigger growth, proliferation or survival of cultured cells relative to untreated control cells.

As used herein, the term 'mutein' is synonymous with 'mutant' as well as 'variant' and refers to functional mutein of the thermostable FGF10 polypeptide carrying the stabilizing amino acid substitutions according to the invention and having desired biological activity. Furthermore, it refers to functional muteins of the thermostable FGF10 polypeptide carrying additional substitutions (point substitutions, point insertion, and point deletion) when having at least 85% sequence identity to SEQ ID NO: 3 or SEQ ID NO: 5. Thus, functional FGF10 polypeptides carry the stabilizing amino acid substitution of the present invention, and at least 85% or more of the amino acids of the SEQ ID NO: 3 or SEQ ID NO: 5. A person skilled in the art is fully aware of amino acid substitutions that are either less likely or not likely to significantly affect polypeptide function (e.g., replacing one aliphatic amino acid with a second aliphatic amino acid). In general, it is possible to replace residues which form the tertiary structure, provided that residues performing a similar function are used. For example, the 15% of sequence variability is preferably represented by conservative substitutions of L-amino acids, wherein the newly inserted amino acid has similar physicochemical properties as the original substituted amino acid, and which, according to state-of-the-art knowledge, is expected to have no or minimal impact on protein structure, activity, and stability. Examples of conservative substitutions known to those skilled in the art include the substitution of one hydrophobic residue for another, or the substitution of one charged or polar residue for another. Such conservative substitutions include amino acid substitution within each of the following groups: (i) glycine, alanine, valine, leucine, and isoleucine; (ii) phenylalanine, tyrosine, and tryptophan; (iii) serine and threonine; (iv) aspartic acid, and glutamic acid; (v) glutamine and asparagine; and (vi) lysine, arginine, and histidine. Further guidance concerning which amino acid changes are likely to be phenotypically silent (i.e., are not likely to have a significant deleterious effect on a protein function) can be found in Bowie, J.U. et al., 1990, 'Deciphering the Message in Protein Sequences: Tolerance to Amino Acid Substitutions', Science 247:1306-1310). In other instances, the type of residue may be completely unimportant if the alterations occur at noncritical regions of the protein (i.e., the protein regions that are not critical for protein function).

As used herein, the term 'sequence identity' refers to a relationship between two polypeptide sequences and is defined by the percentage of positional sequence identity over the full length of the reference amino acid sequence. The percentage of positional sequence identity determines the number of exactly same residues in the identical positions over a defined length in a given alignment multiplied by 100. Two polypeptides have '100% sequence identity' if the amino acid residues of the two polypeptides are the same when aligned for maximal correspondence. Bioinformatics methods of sequence alignment are well known in the art. Percentage of positional sequence identity can be calculated using standard software programs. Preferred computer programs include, but are not limited to, BLASTP, MatGAT, Water (EMBOSS), Matcher (EMBOSS), LALIGN. The well-known Smith-Waterman algorithm can also be used to determine sequence identity.

The amino acid sequence of the thermostable FGF10 polypeptide according to the invention may be identical to the reference sequence of FGF10 polypeptide (SEQ ID NO: 3 or SEQ ID NO: 5), i.e., it may have 100% sequence identity, or it may contain up to certain integer number of amino acid substitutions (corresponding to max. 15% sequence variability) compared to the reference sequence, so the sequence identity of FGF10 polypeptide is less than 100%. These amino acid changes contributing to sequence variability may be formed by point substitutions, point insertion, point deletion, or their combination. The changes may occur at the N-terminal or C-terminal parts of the reference polypeptide, or anywhere between these terminal parts of the reference polypeptide, spread either individually between the individual amino acids of the reference polypeptide or in one or more contiguous regions of the reference sequence.

In vertebrate species such as primates, mouse, rats, rabbits, pigs, dogs, cows, horses, falcons, lizards, and human, FGF10 is highly conserved and show at least 85% sequence identity across a wide range of species. A person skilled in the art will understand that 15% of sequence variability (corresponding to 85% sequence identity described herein) of the thermostable FGF10 polypeptide can be achieved, for example, using the FGF10 sequence of non-human origin. A FGF10 protein, according to embodiments of the present invention having at least 85% identity to the thermostable FGF10 polypeptide, is unlikely to include proteins other than those resembling FGF10 since other members of the FGF family generally have much lower sequence identity.

FGF10 polypeptides derived from human FGF10, according to the invention, can be prepared by gene synthesis or mutagenesis. In general, the gene encoding the FGF10 polypeptide is cloned into an expression vector and subsequently expressed in transformed host organisms, preferably microorganisms. The host organism expresses the foreign gene to produce FGF10 polypeptide under the given expression conditions. The gene encoding the FGF10 polypeptide can also be prepared in eukaryotes, such as yeast or mammalian cells.

An example of the nucleotide sequence of recombinant FGF10-wt (5'-3') with upstream sequences in pET28b vector is shown below (SEQ ID NO: 18). The start codon atg and the stop codon TAA are marked in gray, the N-terminal His-tag (6 × His) is marked in bold and underlined, the thrombin cleavage recognition site is marked in black and the restriction sites for NcoI, NdeI, and XhoI allowing cloning into the expression vector pET28b are underlined (ccatgg - 5 'NcoI, CATATG - 5' NdeI and CTCGAG - 3 'XhoI). The encoding sequence of FGF10 polypeptide begins with a start codon atg and ends with a stop codon TAA.

ccatgggcagcagccatcatcatcatcatcacagcagcggcctggtgccgcgcggcagcCAT
ATGCTGGGTCAAGATATGGTTAGTCCGGAAGCAACCAATAGCAGCAGCAGCTCATTTAGCAG
CCCGAGCAGCGCAGGTCGTCATGTTCGTAGCTATAATCATCTGCAGGGTGATGTTCGTTGGC
GTAAACTGTTTAGCTTTACCAAATACTTCCTGAAAATCGAAAAGAACGGTAAAGTTAGCGGC
ACCAAAAAAGAAAATTGCCCGTATAGCATTCTGGAAATTACCAGCGTTGAAATTGGTGTTGT
TGCCGTGAAAGCAATCAACAGCAACTATTATCTGGCCATGAACAAAAAAGGCAAACTGTATG
GTAGCAAAGAGTTCAACAACGACTGCAAACTGAAAGAACGCATTGAAGAAAATGGCTATAAT
ACCTATGCCAGCTTTAACTGGCAGCATAATGGTCGTCAGATGTATGTTGCACTGAATGGTAA
AGGTGCACCGCGTCGTGGTCAGAAAACCCGTCGTAAAAATACCAGCGCACATTTTCTGCCGA
TGGTTGTTCATAGCTAATAGCTCGAG (SEQ ID NO: 18)

FGF10 polypeptide used for insertion of amino acid substitutions according to the invention can be isolated from any of vertebrate species such as primates, mouse, rats, rabbits, pigs, dogs, cows, horses, falcons, snakes, lizards, and human. Preferably, the FGF10 polypeptide is derived from a human source. However, any biologically active forms (i.e., variants) of mammalian, avian, or reptile FGF10 polypeptides with at least 85%, or 86%, 87%, 88%, 89%, 90%, 91%, 92%, 93%, 94%, 95%, 96%, 97%, 98%, 99% and 100% sequence identity to the amino acid sequence of human FGF10 (SEQ ID NO: 1), or the thermostable FGF10 polypeptide (SEQ ID NO: 3, SEQ ID NO: 5) may be used.

### BRIEF DESCRIPTION OF FIGURES

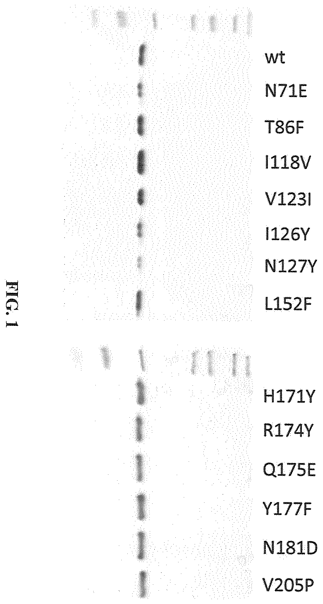

**FIGURE 1****.** shows the SDS-PAGE gels of purified FGF10-wt and its single-points mutants (carrying one of the following substitutions N71E, T86F, I118V, V123I, I126Y, N127Y, L152F, H171Y, R174Y, Q175E, Y177F, N181D, V205P). Protein marker: 116, 66.2, 45, 35, 25, 18.4, 14.4 kDa. Recombinant FGF10-wt as well as its mutants with N-terminal His-tag and thrombin cleavage site have M_{w} of ~ 21.4 kDa.
**FIGURE 2****.** shows proliferation of lung epithelial 4MBr-5 cells induced by FGF10-wt and its single-point mutants with enhanced stability. Determined ED₅₀ values for this effect for FGF10-wt and its single point-mutants carrying the amino acid substitutions V123I, L152F, Q175E, and N181D were 0.8, 0.4, 1.4, 0.2, and 1.6 ng/mL, respectively. Each data point represents mean value from triplicate samples. The error bars were smaller than the symbol size, generally less than 4%.

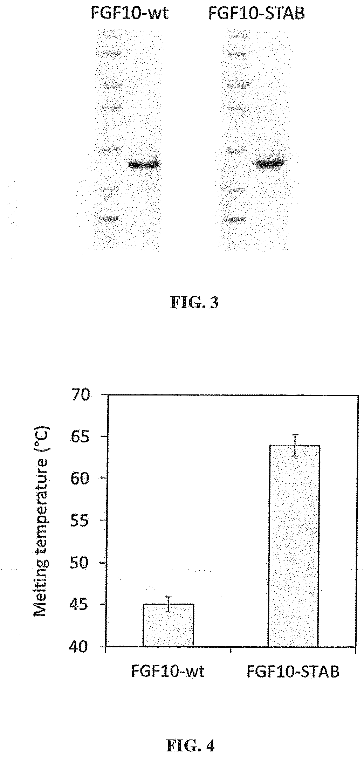

**FIGURE 3****.** shows the SDS-PAGE gels of purified FGF10-wt and its 4-point mutant with enhanced stability FGF10-STAB. Protein marker: 116, 66.2, 45, 35, 25, 18.4, 14.4 kDa. Recombinant FGF10-wt and FGF10-STAB with N-terminal His-tag and thrombin cleavage site have M_{w} of ~ 21.4 kDa.
**FIGURE 4****.** shows the comparison of thermostability of FGF10-wt with its 4-point mutant FGF10-STAB. Melting temperature (*T*ₘ) was determined using DSF.

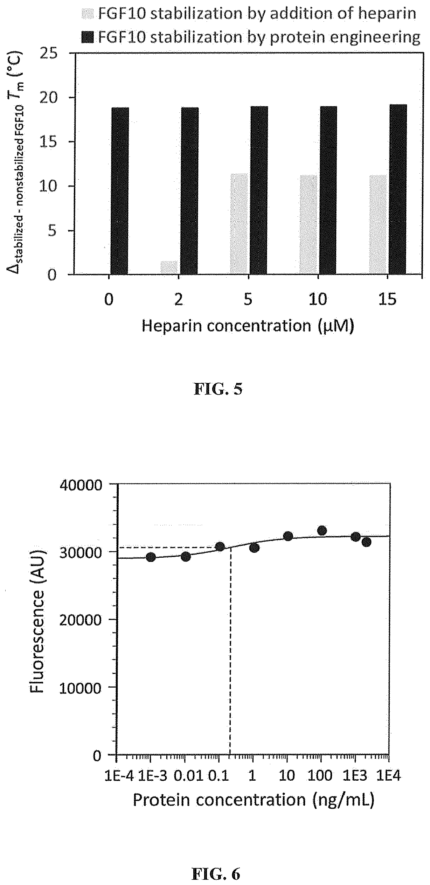

**FIGURE 5****.** shows the comparison of the extent of FGF10 polypeptide stabilization mediated by protein engineering via introduction of four stabilizing amino acid substitutions (V123I+L152F+Q175E+N181D) with the polypeptide stabilization mediated by heparin addition. The protein stabilization is expressed as a difference in melting temperature (Δ*T*ₘ,) between stabilized and unstabilized (wild-type polypeptide) FGF10 polypeptide. *T*ₘ was determined using DSF.
**FIGURE 6****.** shows proliferation of lung epithelial 4MBr-5 cells induced by FGF10-STAB. Determined ED₅₀ values for this effect was 0.2 ng/mL which is in agreement with expected ED₅₀ value < 15 ng/mL for recombinant FGF10-wt. Each data point represents mean value from triplicate samples. The error bars were smaller than the symbol size, generally less than 4%.
**FIGURE 7****.** shows the ability of FGF10-STAB to retain its biological activity during prolonged incubation at 37°C. (A) FGF10-wt and FGF10-STAB were preincubated in complete medium for 10 min, 30 min, 1h, 2 h, 4 h, 6 h, 8 h, 12 h, 14 h, and 24 h prior treatment of the cells. Mammary adenocarcinoma MCF7 cells endogenously expressing FGFR2 receptor were cultivated in complete medium for 24 hours. Then, the cells were stimulated by FGF10-wt or FGF10-STAB for 30 minutes and immunoblotted for phosphorylated ERK1/2. FGF10 levels in media aliquots were used as loading controls. While the biological activity of wild type FGF10 declined with time of heat-preincubation, the thermostabilized FGF10-STAB retained full biological activity even after 24 h at 37°C. (B) Densitometric analysis of Western blots performed using ImageJ software. (C) Densitometric analysis of loading control. Error bars represent the standard error of the mean from three independent experiments. Student's t-test, ^{∗∗∗}p<0.001, ^{∗∗}p<0.01, ^{∗}p<0.05.

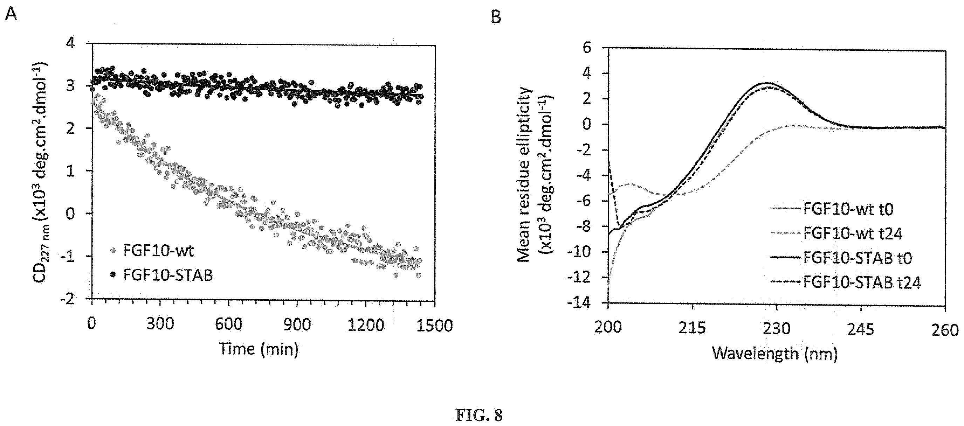

**FIGURE 8****.** shows the ability of FGF10-STAB to retain its secondary structure during prolonged incubation at 37°C. Changes in the secondary structure of both proteins were monitored by CD spectroscopy. (A) The structural integrity of FGF10-wt and FGF10-STAB of concentration 0.2 mg/mL in 25 mM phosphate buffer (pH 7.5) containing 750 mM NaCl was followed by monitoring the ellipticity over the wavelength range of 200-260 nm at 37°C for 24 h (the maximum time of measurement was limited by the capacity of the bomb with compressed nitrogen used in the CD spectroscopy protocol). While FGF10-STAB exhibited preserved secondary structure for 24 hr at 37°C, determined half-life of the secondary structure of FGF10-wt, evaluated at 227 nm, was 11.6 hr. (B) CD spectra of FGF10-wt and FGF10-STAB recorded before and after protein incubation at 37°C for 24 hr. Correctly folded FGF10 protein samples exhibited CD spectra with a broad positive peak centered near 227 nm and a minimum at around 204 nm, characteristic for β-rich proteins of β-II type. While FGF10-STAB after incubation at 37°C for 24 hr exhibited identical shape and intensity of the CD spectrum as that before the heat incubation, FGF10-wt during incubation at 37°C undergoes conformation change which is reflected by different shape and intensity of the CD spectrum when compared with the spectrum of FGF10-wt recorded before the incubation.

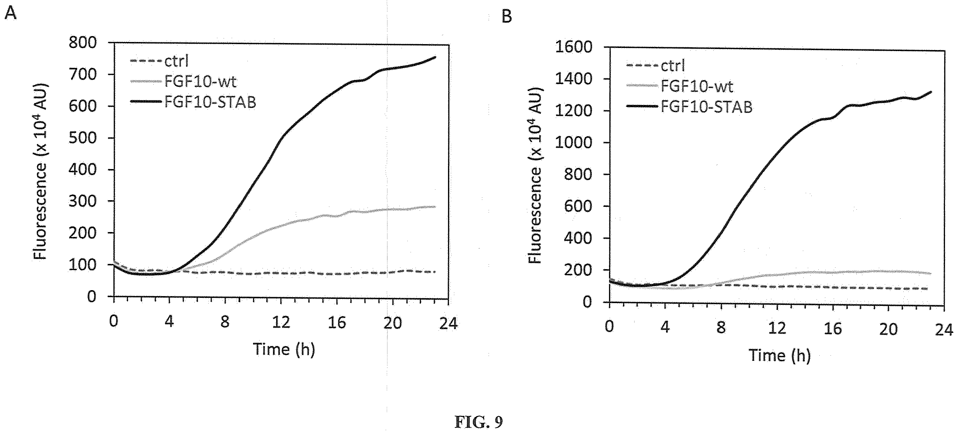

**FIGURE 9****.** shows the ability of FGF10-STAB to induce sustained activation of FGFR signaling in MCF7 cells transfected by TR01G vector carrying the pKrox24(MapERK)-DsRed reporter. (A) Transactivation of pKrox24 reporter in MCF7 cells induced by stimulation of fresh FGF10-wt and FGF10-STAB. (B) Transactivation of pKrox24 reporter in MCF7 cells induced by stimulation of FGF10-wt and FGF10-STAB after the sample preincubation at 37°C for 2 hr. The FGF-mediated pKrox24 induction was monitored by live cell imaging of DsRed fluorescence over 24 hr.

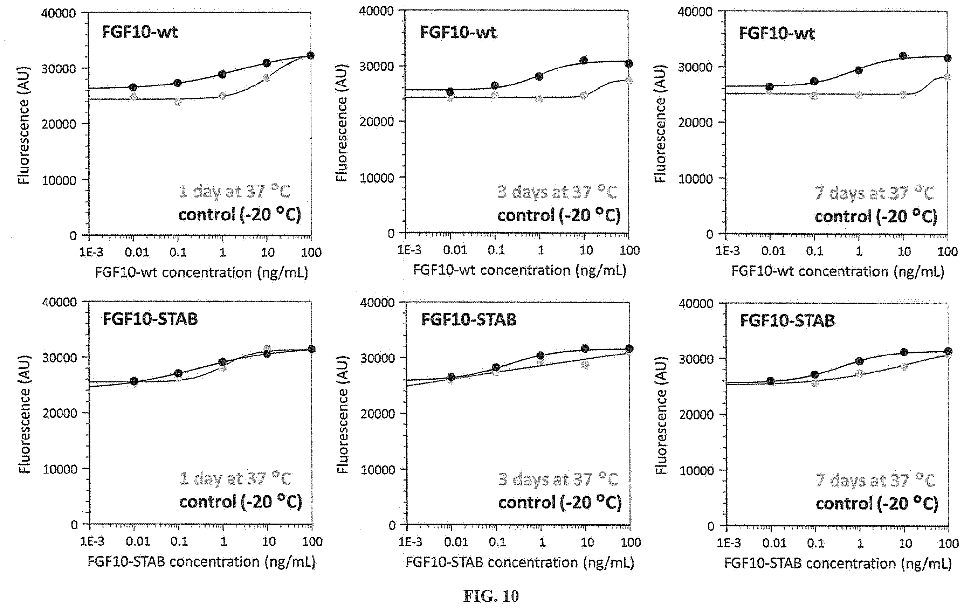

**FIGURE 10****.** demonstrates FGF10-STAB's ability to induce proliferation of lung epithelial 4MBr-5 cells after 7-days of incubation at 37°C. FGF10-wt and FGF10-STAB were exposed to 37°C for 1, 3, and 7 days and then used to treat the 4MBr-5 cells. The plots show resorufin fluorescence, measured after 3 days of cultivation with FGF10 samples. Each data point represents mean value from triplicate samples. The error bars were smaller than the symbol size, generally less than 4%. While FGF10-STAB retains its ability to induce the cell proliferation after the whole preincubation period, the FGF10-wt ability to induce the cell proliferation decrease already after 1 day of incubation at 37°C.

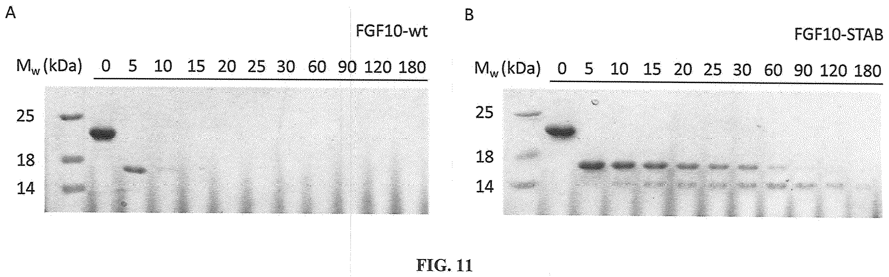

**FIGURE 11****.** shows comparison of FGF10-wt and FGF10-STAB resistance to proteolytic trypsin degradation. (A) FGF10-wt and (B) FGF10-STAB were digested at 37°C by trypsin (in a final concentration of 2 µg protease/100 µg FGF10) for 180 minutes. Products of the reaction were visualized by SDS-PAGE. The difference between time 0 and 5 minutes is most probably caused by cleavage of N-terminal part of recombinant FGF10 polypeptide (formed by N-terminal His-tag, thrombin cleavage site, and the following 29 amino acids) which does not adversely affect the biological function of FGF10.

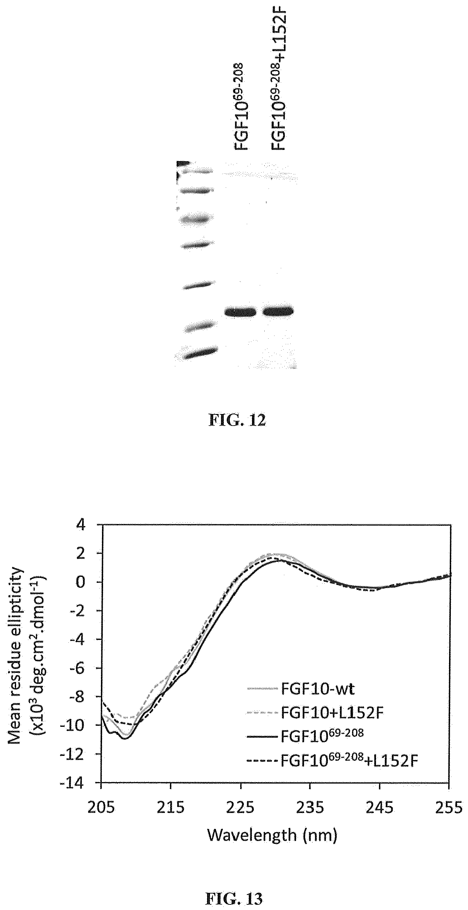

**FIGURE 12****.** shows the SDS-PAGE gels of purified FGF10 polypeptide consisting of amino acid sequence Ser69 - Ser208 of SEQ ID NO: 1 (FGF10⁶⁹⁻²⁰⁸) and its stable variant carrying the substitution L152F (FGF10⁶⁹⁻²⁰⁸+L152F). Protein marker: 116, 66.2, 45, 35, 25, 18.4, 14.4 kDa. Recombinant FGF10⁶⁹⁻²⁰⁸ and FGF10⁶⁹⁻²⁰⁸+L152F with N-terminal His-tag and thrombin cleavage site have M_{w} of ~ 18.5 kDa.
**FIGURE 13****.** show CD spectra of FGF10 polypeptides demonstrating correct folding of FGF10 polypeptide consisting of amino acid sequence Ser69 - Ser208 (FGF10⁶⁹⁻²¹¹) and its stable variant carrying the substitution L152F (FGF10⁶⁹⁻²⁰⁸+L152F).

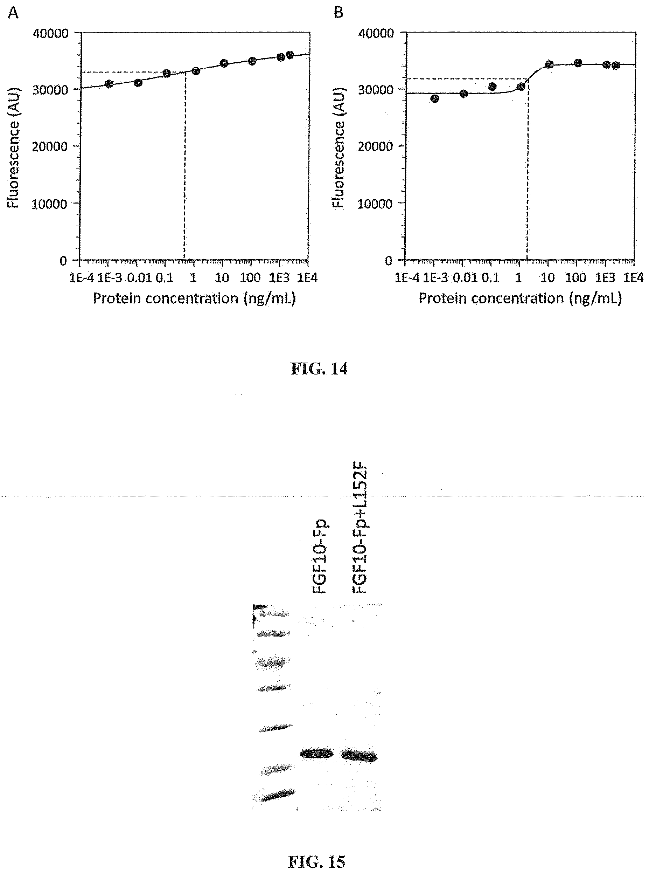

**FIGURE 14****.** shows proliferation of lung epithelial 4MBr-5 cells induced by (A) FGF10 polypeptide consisting of amino acid sequence Ser69 - Ser208 (FGF10⁶⁹⁻²⁰⁸) and (B) its stable variant carrying the substitution L152F (FGF10⁶⁹⁻²⁰⁸+L152F). Determined ED₅₀ values for this effect for FGF10⁶⁹⁻²⁰⁸ and FGF10⁶⁹⁻²⁰⁸+L152F were 0.5 and 1.9 ng/mL, respectively. Each data point represents mean value from triplicate samples. The error bars were smaller than the symbol size, generally less than 4%.
**FIGURE 15****.** shows the SDS-PAGE gels of purified FGF10 polypeptide having 88% sequence identity to SEQ ID NO: 3 or SEQ ID NO: 5, which corresponds to FGF10 from *Falco peregrinus* (FGF10-Fp) and its stable variant carrying the substitution L152F (FGF10-Fp+L152F). Protein marker: 116,66.2,45,35,25, 18.4, 14.4 kDa. Recombinant FGF10-Fp and FGF10-Fp+L152F with N-terminal His-tag and thrombin cleavage site have M_{w} of ~ 17.9 kDa.

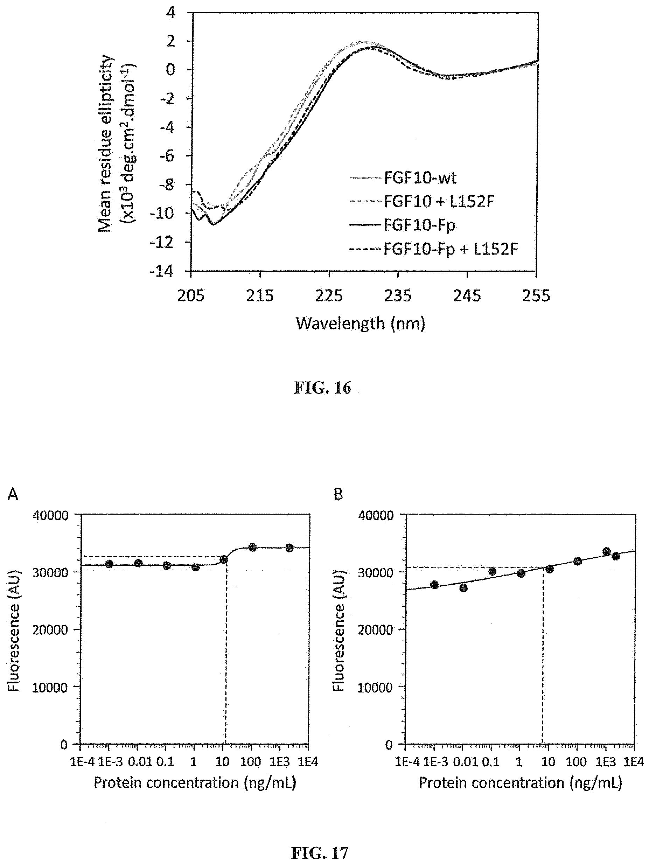

**FIGURE 16****.** represents CD spectra of FGF10 polypeptides demonstrating that introduction of 12% variability (i.e., FGF10-Fp with 88% sequence identity to SEQ ID NO: 3 or SEQ ID NO: 5) into the FGF10 amino acid sequence does not alter the protein secondary structure. Simultaneously the introduction of single-point stabilizing substitution L152F into the FGF10 polypeptide with 88% sequence identity to SEQ ID NO: 3 or SEQ ID NO: 5 (FGF10-Fp+L152F) does not alter the protein secondary structure.
**FIGURE 17****.** shows proliferation of lung epithelial 4MBr-5 cells induced by (A) FGF10 polypeptide having 88% sequence identity to SEQ ID NO: 3 or SEQ ID NO: 5 corresponding to FGF10 from *Falco peregrinus* (FGF10-Fp) and (B) its stable variant carrying the substitution L152F (FGF10-Fp+L152F). Determined ED₅₀ values for this effect for FGF10-Fp and FGF10-Fp+L152F were 12.7 and 6.1 ng/mL, respectively. Each data point represents mean value from triplicate samples. The error bars were smaller than the symbol size, generally less than 4%.

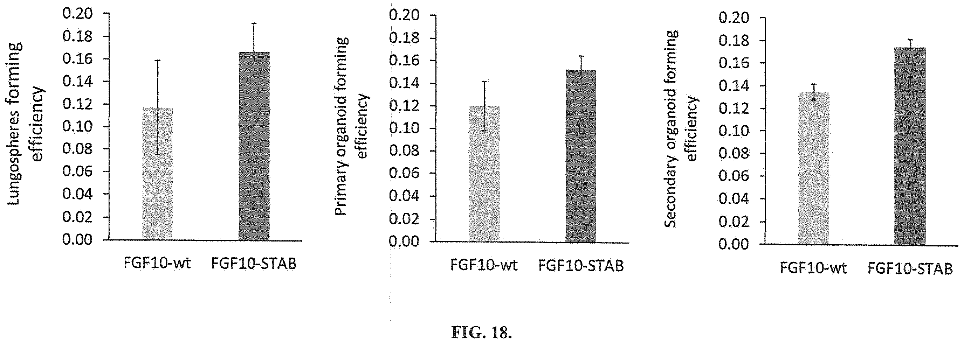

**FIGURE 18****.** shows higher lungospheres and lung organoid forming efficiency of FGF10-STAB when compared with FGF10-wt. The plot shows mean ± SD.

### EXAMPLES

The following examples are presented in order to illustrate the embodiments of the present invention. Examples given are illustrative in nature only and not intended to be limiting. Although methods and materials similar or equivalent to those described herein can be used in the testing of the present invention, suitable methods and materials are described below.

### EXAMPLE 1. In silico design of potentially stabilizing single-point mutations in FGF10 by energy-based and evolution-based approaches

Available crystal structure of FGF10-FGFR2b complex (PDB ID 1NUN) with a resolution of 2.9 Å was downloaded from RCSB PDB database and used for prediction of potentially stabilizing substitutions in FGF10 by energy-based approach. The complex structure was prepared for analyses by removing all ligands and water molecules and by creating the pdb file containing separated chain A corresponding to FGF10 molecule and containing amino acid residues Ser69 - His207. Protein side chains were minimized and scored to determine whether minimization passed correctly. Stability effects of all possible single-point mutations were estimated using the force-field calculations. ΔΔG free energies were collected over all 20 mutations in a particular position. Evolutionary conservation was estimated using phylogenetic analysis of homologous sequences. Mutations with ΔΔG < -1.0 kcal/mol and conservation ≤ 7 were selected for further analysis. Functionally relevant positions, such as residues located in receptor- and heparin-binding sites, were excluded from the selection in order to minimize the occurrence of mutations compromising protein function.

For the prediction of potentially stabilizing substitutions in FGF10 by evolution-based approach, multiple sequence alignment of FGF10 with related proteins was constructed. The FGF10 protein sequence (SEQ ID NO: 1) was used as a query for PSI-BLAST (Altschul, T.S. et al., 1997, Nucleic Acids Res. 25:3389-3402) search against nr database of NCBI. Sequences with e-value of 10⁻¹⁵ collected after 3 iterations of PSI-BLAST were clustered by CD-HIT (Li, W. & Godzik, A., 2006, Bioinformatics 22:1658-1659) at the 90% identity threshold. The resulting dataset of more than 200 sequences was clustered with CLANS (Frickey, T. & Lupas, A.N., 2004, Bioinformatics 20:3702-3704) using default parameters and varying P-value thresholds. Sequences clustered together with FGF10 at the P-value of 10⁻²⁹ were extracted and aligned with the MUSCLE program (Edgar, R.C., 2004, BMC Bioinformatics 5:113). The final alignment comprising 129 sequences was used as an input for back-to-consensus analysis using the simple consensus approach. The analysis was performed using the consensus cut-off of 0.5, meaning that a given residue must be present at a given position in at least 50% of all analysed sequences to be assigned as the consensus residue. Mutations identified by consensus have to pass cut-off of ΔΔG < 0.5 kcal/mol evaluated by the force-field calculation.

A list of the best fourteen mutations identified by both energy-based and evolution-based approaches are summarized in Table 1. All identified fourteen mutations were selected for experimental validation. The amino acid numbering corresponds to the sequence of human FGF10 polypeptide (SEQ ID NO: 1).

**Table 1. The stabilizing mutations identified in FGF10 by energy-based and evolution-based predictions. Mutations identified by evolution-based approach are highlighted in bold.**

| **Mutation** | **ΔΔG (kcal/mol)** | **Conservation** | **Consensus** | **Amino acids in MSA** |
|---|---|---|---|---|
| N71E | -2.331 | 5 | - | D,G,E,K,Y,N,F,S,P,V,H |
| T86F | -1.783 | 7 | - | H,N,P,T,Q |
| I126Y | -3.167 | 7 | - | I,F,M,L,V |
| N127Y | -2.637 | 3 | - | G,A,E,Y,K,N,F,Q,T,S,H |
| S143P | -2.089 | 7 | - | K,V,A,E,T,S |
| H171Y | -1.996 | 5 | - | Q,R,K,H,N,P,E |
| R174Y | -3.650 | 6 | - | G,E,H,K,R,T,Q |
| Q175E | -2.102 | 6 | - | Q,A,E,P,H,R |
| N181D | -2.100 | 7 | - | N,R,G,D,M,S |
| V205P | -1.377 | 2 | - | P,V,F,I,Q,M,T,A,E,K,N,L |
| **I118V** | 0.008 | 8 | 0.69 | M,T,Q,I,E,V |
| **V123I** | -1.590 | 8 | 0.83 | L,I,V |
| **L152F** | 0.395 | 9 | 0.62 | L,F |
| **Y177F** | -0.100 | 8 | 0.83 | Y,F |

| | | | | |
|---|---|---|---|---|
| AAG: predicted change in Gibs free energy; MSA: multiple sequence alighment. | | | | |

### EXAMPLE 2. Construction of fourteen single point mutants of FGF10 and their expression and purification to homogeneity by affinity chromatography

Mutant recombinant genes were constructed using OneClick method and Phusion polymerase (New England Biolabs) according to the instructions of the web program OneClick and recommendations of the manufacturer of the polymerase. Mutagenesis of FGF10 was done by whole plasmid PCR using the vector pET28b-His-thrombin:*fgf10* as a template and two reverse-complement oligonucleotides carrying desired mutations. Resulting constructs were transformed into *Escherichia coli* NEB5-alpha competent cells. Cells were plated on agar plates with kanamycin (50 µg/mL) and grown overnight at 37°C. Plasmids were isolated and nucleotide sequences were confirmed by commercial sequencing. *E. coli* BL21(DE3) cells were transformed with expression vectors, plated on agar plates with kanamycin and grown overnight at 37°C. Single colonies were used to inoculate 10 mL of LB medium with kanamycin (50 µg/mL) and cells were grown overnight at 37°C. Overnight culture was used to inoculate 1 L of LB medium with kanamycin. Cells were cultivated at 37°C. The expression was induced with isopropyl b-D-1-thiogalactopyranoside (IPTG) to a final concentration of 0.5 mM. Cells were then cultivated overnight at 20°C. At the end of cultivation, biomass was harvested by centrifugation and washed by buffer (20 mM di-potassium hydrogen phosphate and potassium dihydrogen phosphate, pH 7.5, 0,5 M NaCl, 10 mM imidazole). Cells in suspension were disrupted by sonication and cell lysate was centrifuged. Proteins were purified from crude extracts using single-step nickel affinity chromatography. The presence of proteins in peak fractions was demostrated by SDS-PAGE in 15% polyacrylamide gel. Purified proteins were dialyzed against the phosphate buffer containing 750 mM NaCl. Purification of FGF10 mutants by affinity chromatography resulted in homogeneous proteins with purity higher than 95% as verified by SDS-PAGE electrophoresis (Figure 1). The yield of majority of FGF10 single-point mutants ranged from 10 to 21 mg per litre of cell culture. The only exception was FGF10 mutation S143P, which caused a decrease in the protein yield of about 90%. Therefore, this mutation was excluded from further analysis.

### EXAMPLE 3. Determination of thermostability of single-point FGF10 mutants

The thermostability of single-point FGF10 mutants predicted by energy-based and evolution-based approaches was determined by differential scanning fluorimetry (DSF) and differential scanning calorimetry (DSC). Thermal unfolding of 0.2 mg/mL and 1.0 mg/mL protein solutions in 20 mM phosphate buffer pH 7.5 containing 750 mM sodium chloride was followed by monitoring changes in the intrinsic fluorescence and the heat capacity by using the NanoTemper Prometheus DSF system and the VP-capillary DSC system, respectively. The measurements were performed at the temperatures from 25 to 90°C at 1 °C/min heating rate. The melting temperatures (*T*ₘ) were evaluated as a midpoint of normalized fluorescence thermal transitions or as a maximum of apparent heat capacity peaks in the case of DSF and DSC experiments, respectively. Results are summarized in Table 2. Single-point mutants carrying stability-increasing mutations with Δ*T*ₘ ≥ 2°C were selected for biological activity testing (see Example 4) and upon verification of their unmodified activity, the stabilizing mutations were combined, employing free energy calculations, into multiple-point mutants (see Example 5).

**Table 2. Thermostability of FGF10 mutants determined by differential scanning fluorimetry (DSF) and differential scanning calorimetry (DSC). Mutations selected for construction of combined 4-point mutant are highlighted in bold.**

| **Mutant** | **DSF *T*ₘ (°C)¹** | **DSF** Δ*T*ₘ **(°C)** | **DSC *T*ₘ (°C)¹** | **DSC Δ*T*ₘ (°C)** |
|---|---|---|---|---|
| FGF10-wt | 45.3 | - | 46.4 | - |
| N71E | 44.8 | -0.5 | 46.0 | -0.4 |
| T86F | 43.8 | -1.5 | 45.4 | -1.0 |
| I118V | 45.9 | 0.6 | 46.1 | -0.3 |
| **V123I** | **48.7** | **3.4** | **49.3** | **2.9** |
| I126Y | 40.7 | -4.6 | 42.7 | -3.7 |
| N127Y | 46.7 | 1.4 | 47.8 | 1.4 |
| **L152F** | **52.5** | **7.2** | **53.5** | **7.1** |
| H171Y | 45.3 | 0.0 | 46.8 | 0.4 |
| R174Y | 41.0 | -4.3 | 42.2 | -4.2 |
| **Q175E** | **49.1** | **3.8** | **50.5** | **4.1** |
| Y177F | 45.1 | -0.2 | 46.4 | 0.0 |
| **N181D** | **48.0** | **2.7** | **49.7** | **3.3** |
| V205P | 47.2 | 1.9 | 48.4 | 2.0 |

| | | | | |
|---|---|---|---|---|
| *T*ₘ: melting temperature; Δ*T*ₘ: change in melting temperature upon introduced mutation; ¹The average from three independent experiments is presented (standard deviations were less than 4%). | | | | |

### EXAMPLE 4. Biological activity testing of single-point FGF10 mutants with enhanced stability using lung epithelial 4MBr-5 cell proliferation assay

FGF10 is expressed in mesenchymal cells and facilitates epithelial-mesenchymal signaling through binding the epithelially expressed FGF receptor 2b (FGFR2b). 4MBr-5 rhesus monkey bronchial epithelial cells require EGF to proliferate, but since they naturally express FGFR2b, FGF10 may be used to induce the cell proliferation instead. Activation of FGFR2b signaling results in a mitogenic response, which can be quantified using various cell proliferation/viability assays, such as resazurin-based assay. Resazurin is a cell-permeable redox indicator and reflects the cell viability by converting from a nonfluorescent blue dye resazurin to the highly fluorescent red dye resorufin in response to the chemical reduction of growth medium resulting from cell growth. The fluorescent signal is proportional to the number of living cells in the sample.

To test biological activity of FGF10 variants with enhanced thermodynamic stability, 4MBr-5 cells were seeded in 96-well plates at 7.5 × 10³ cells per well in 150 µL of 4MBr-5 starvation media (Ham's F-12K - Gibco, 10% Newborn Calf Serum, 1% PenicillinG/Streptomycin Sulfate, 2 µg/mL heparin). Protein treatment was administered to the cells 3 hours after seeding, to allow proper adhesion of cells. FGF10 variants were serially diluted in starvation media and 50 µL of protein solution were added to the wells. Final concentrations of FGF10 variants ranged from 0,001 ng/mL to 2000 ng/mL, each concentration was tested in triplicate. Mouse EGF was used as a positive control. Following 3 days of cultivation, 20 µl of resazurin solution were added to each well and the plates were incubated overnight. Measurement of metabolic activity was performed by fluorescence reading (560 nm excitation, 590 nm emission) on Synergy 4H reader (BioTek, USA). Cellular proliferation caused by FGF10 is represented by median effective dose ED₅₀ (concentration of protein needed to achieve half of the maximal proliferation) calculated by online software ED₅₀ Calculator (AAT Bioquest, USA). FGF10-wt, as well as its thermostable single-point variants, effectively induced proliferation of 4MBr-5 cells (Figure 2). Comparison of median effective dose (ED₅₀) revealed that all thermostable single-point variants are similarly effective in inducing the proliferation of 4MBr-5 cells (ED₅₀ values ranging from 0.2 to 1.6 ng/mL) as FGF10-wt (ED₅₀ = 0.8 ng/mL). The expected ED₅₀ value of commercially available recombinant human FGF10 molecules measured by a cell proliferation assay using 4MBr-5 cells is < 15 ng/mL. This example demonstrates unmodified biological activity of thermostable single-point variants of FGF10.

### EXAMPLE 5. In silico verification of multiple-point mutants of FGF10 polypeptide

All previously selected single-point substitutions that have been experimentally confirmed as stabilizing while preserving unmodified biological activity were *in silico* combined into all possible two-point and multiple-point mutants and their energies were estimated using the force-field calculations. Both two-point and multiple-point mutants were *in silico* designed using protocol 16 incorporating the backbone flexibility within the ddg monomer module of Rosetta software according to Kellogg et al. (Kellogg, E. et al., 2011, Proteins, 79:830-838). The soft-repulsive design energy function was used for side chains repacking. Optimization was performed on whole protein without distance restriction. Three rounds of optimization with increasing weight on the repulsive term were performed. A minimum energy from 50 iterations was employed as a final parameter describing the stability effect. The calculated free energies of multiple-point mutants and their comparison with the expected ΔΔG values estimated as the sum of contributions from the individual stabilizing mutations are summarized in Table 3. The observed difference between the calculated ΔΔG of the multiple-point mutants and sum of ΔΔG of the individual stabilizing mutations suggested that individual mutations do not have significantly antagonistic effect on each other and their combination will lead to stable FGF10 polypeptides.

**Table 3. In silico prediction of stabilizing effect of multiple-point mutants of FGF10 polypeptide.**

| **Mutations** | **Sum of single-point mutant contribution ΔΔG (kcal/mol)** | **Calculated values for multiple-point mutants ΔΔG (kcal/mol)** |
|---|---|---|
| V123I + L152F | -1.2 | -4.3 |
| V123I + Q175E | -3.7 | -3.3 |
| V123I+ N181D | -3.7 | -1.8 |
| L152F + Q175E | -1.7 | -2.2 |
| L152F + N181D | -1.7 | -0.8 |
| Q175E + N181D | -4.2 | -0.5 |
| V123I + L152F + Q175E | -3.3 | -5.2 |
| V123I + L152F + N181D | -3.3 | -3.3 |
| V123I +Q175E + N181D | -5.8 | -2.7 |
| L152F + Q175E + N181D | -3.8 | -3.7 |
| V123I+L152F+Q175E+N181D | -5.4 | -5.0 |

| | | |
|---|---|---|
| ΔΔG: predicted change in Gibs free energy. | | |

### EXAMPLE 6. Construction, purification, and thermostability testing of 4-point FGF10 mutant

The gene encoding 4-point mutant of FGF10, FGF10-STAB, was commercially synthetized and subcloned in the *Ndel* and *Xhol* sites of the expression vector pET28b-His-thrombin under the control of the T7lac promoter. Resulting construct was transformed into *E. coli* NEB5-alpha competent cells. The cells were plated on agar plates with kanamycin (50 µg/mL) and grown overnight at 37°C. Plasmids were isolated and nucleotide sequences were confirmed by commercial sequencing. *E. coli* BL21(DE3) cells were transformed with expression vector, plated on agar plates with kanamycin and grown overnight at 37°C. Single colony was used to inoculate 10 mL of LB medium with kanamycin (50 µg/mL) and cells were grown overnight at 37°C. The overnight culture was used to inoculate 1 L of LB medium with kanamycin. Cells were cultivated at 37°C. The expression was induced with IPTG to a final concentration of 0.5 mM. Cells were then cultivated overnight at 20°C. At the end of cultivation, biomass was harvested by centrifugation, washed with purification buffer (20 mM di-potassium hydrogen phosphate and potassium dihydrogen phosphate, pH 7.5, 0,5 M NaCl, 10 mM imidazole), and wet biomass was frozen at -80°C. Defrosted cultures were disrupted by sonication and lysate was centrifuged. FGF10-STAB was purified from crude extracts using single-step nickel affinity chromatography. Purified protein was dialyzed against buffer containing 750 mM NaCl. The protein purity was proved by SDS-PAGE in 15% polyacrylamide gel (Figure 3). An average yield of FGF10-STAB was 12 mg per litre of cell culture. Thermostability of FGF10-STAB was determined by DSF and DSC upon dilution of the protein solution to 1.0 mg/mL. The measurements were performed at the temperatures from 25 to 90°C at 1 °C/min heating rate. The melting temperatures (*T*ₘ) were evaluated as a midpoint of normalized fluorescence thermal transitions or as a maximum of apparent heat capacity peaks in the case of DSF and DSC experiments, respectively. FGF10-STAB exhibits *T*ₘ 64°C which corresponds to the increase in the melting temperature by 19°C when compared with FGF10-wt (Figure 4).

### EXAMPLE 7. Stabilization of FGF10 polypeptide by protein engineering surpass the stabilization effect of heparin

Stabilizing effect of heparin addition has been previously reported for different members of FGF protein family including FGF10 polypeptide. The effect of heparin on thermal stability of stabilized form of FGF10 polypeptide, FGF10-STAB, was therefore tested and compared with the stabilizing effect of heparin on FGF10-wt. Melting temperature of FGF10-STAB and FGF10-wt at a range of heparin concentrations was determined by DSF upon dilution of the protein solution to 1.0 mg/mL. The measurements were performed at the temperatures from 25 to 90°C at 1 °C/min heating rate. The *T*ₘ was evaluated as a midpoint of normalized fluorescence thermal transitions. While the melting temperature of FGF10-wt (*T*ₘ 46°C; determined in the absence of heparin) was significantly increased in the presence of 5-15 µM heparin (*T*ₘ 57°C), the thermal stability of FGF10-STAB stabilized by protein engineering, was not affected even in the presence of the highest heparin concentration tested (15 µM). The heparin-mediated stabilization of FGF10 polypeptide resulted in an increase of the melting temperature by 10°C, whereas the FGF10 stabilization mediated by protein engineering (i.e., by introduction of 4 stabilizing amino acid substitutions) resulted in an increase of the melting temperature by 19°C (Figure 5). This example demonstrates that stabilization of FGF10 by protein engineering compensates and even surpasses the stabilizing effect of heparin.

### EXAMPLE 8. Biological activity testing of 4-point FGF10 mutant with enhanced stability using lung epithelial 4MBr-5 cell proliferation assay

Biological activity of 4-point FGF10 mutant, FGF10-STAB, was determined by cell proliferation assay using 4MBr-5 cell system (Rubin, J.S. et al., 1989, Proc Natl Acad Sci U S A, 86:802-806). Prior to the analysis, the lyophilized form of FGF10 was reconstituted in water, diluted in 0.1% BSA to a final concentration 100 µg/mL and either immediately used or stored at -20°C. The 4MBr-5 cells were seeded in 96-well plates at 7.5 × 10³ cells per well in 150 µL of 4MBr-5 starvation media (Ham's F-12K, Gibco^{®} + 10% Newborn Calf Serum + 1% PenicillinG/Streptomycin Sulfate + 2 µg/mL heparin). Protein treatment was administered to the cells 3 hours after seeding, to allow proper adhesion of cells. FGF10-STAB was serially diluted in starvation media and 50 µL of protein solution was added to the wells. Final concentrations of FGF10 ranged from 0.001 ng/mL to 2000 ng/mL, each concentration was tested in triplicate. Mouse EGF was used as a positive control. Following 3 days of cultivation, 20 µL of resazurin solution were added to each well and the plates were incubated overnight. Measurement of metabolic activity was performed by fluorescence reading (560 nm excitation, 590 nm emission) on Synergy 4H reader. Cellular proliferation caused by FGF10 is represented by median effective dose ED₅₀ (concentration of protein needed to achieve half of the maximal proliferation) calculated by online software ED₅₀ Calculator. Determined ED₅₀ value for FGF10-STAB, i.e., the concentration of FGF10-STAB that induces the one-half of the maximal response in the proliferation of 4MBr-5 cells was 0.21 ng/mL, which fully correspond with the expected ED₅₀ value (<15 ng/mL) of recombinant FGF10-wt for this effect (Figure 6). This example thus demonstrates the unmodified biological activity of FGF10-STAB.

### EXAMPLE 9. Thermostabilized 4-point FGF10 mutant, FGF10-STAB, maintains its biological activity during prolonged incubation at 37°C due to enhanced structural half-life

Intrinsic instability of FGF proteins limits their biological function, manifesting their failure to activate FGF receptor signal transduction over long periods of time (Buchtova, M. et al., 2015, Cell. Mol. Life Sci., 72: 2445-2459). In order to evaluate how enhanced thermodynamic stability of FGF10-STAB correlates with the ability to maintain cell signaling after prolonged incubation at 37°C, the bioactivity of FGF10-STAB was monitored via activation of ERK pathway using mammary adenocarcinoma MCF7 cell signaling assay. The MCF7 cells endogenously express FGFR2 receptor, which is activated upon interaction with FGF10, together with its downstream effectors including ERK1/2. As the biological activity of FGF10 decreases at 37°C, ERK1/2 phosphorylation (pERK 1/2) declines. MCF7 cells were propagated in complete medium (DMEM with 10% FBS and 1% penicillin/streptomycin; Thermo Fisher Scientific). For the western blot experiments, 200,000 MCF7 cells/well were plated into 24 well plates in 0.5 mL of complete medium and next day stimulated with FGF10 at final concentration of 10 ng/mL for 30 minutes, and lysed in 2x Laemmli sample buffer. For this treatment, FGF10 was diluted at concentration of 0.5 µg/mL in complete medium, and pre-incubated at 37°C for up to 24 hours. The cell lysates were boiled (95°C for 7 min), resolved by SDS-PAGE, transferred on the PDVF membrane, and the pERK 1/2 and ERK 1/2 levels were visualized by antibodies and chemiluminescence. Aliquots of media with FGF10 were collected at each preincubation timepoint, mixed with equal volume of 2x Laemmli sample buffer, boiled and western blotted for FGF10. The following antibodies were used: rabbit monoclonal anti-pERK (4376; Cell Signaling), rabbit polyclonal anti-ERK (9102; Cell Signaling), rabbit polyclonal anti-FGF10 (sc-7917; SCBT), and goat anti-rabbit IgG-HRP (A6667; Sigma-Aldrich). While the biological activity of FGF10-wt declined with time of heat-preincubation, the thermo-stabilized FGF10-STAB mutant retained full biological activity even after 24 hours at 37°C (Figure 7). Simultaneously, the structural integrity of FGF10-wt and its thermostabilized 4-point mutant, FGF10-STAB, during incubation at 37°C was monitored by circular dichroism (CD) spectroscopy. Changes in secondary structure of FGF10-wt and FGF10-STAB of concentration of 0.2 mg/mL in 25 mM phosphate buffer (pH 7.5) containing 750 mM NaCl was followed by monitoring the ellipticity over the wavelength range of 200-260 nm at 37°C. Data were recorded in 5 min intervals with 1 nm bandwidth using a 0.1 cm quartz cuvette containing the protein. Recorded denaturation curves (single exponential function) of tested FGF10 variants were globally fitted to exponential decay curves using Origin2019b software (OriginLab, Northampton, MA). Half-life of FGF10 secondary structure, *t*_{1/2} defined as a time required to reduce the initial value of ellipticity, as a measure of protein secondary structure, to 1/2 of the original value, was evaluated from the collected data as a decay constant (*τ*) using the Equation: *t*_{1/2} = In (2) / λ = *τ* ln (2), where λ is exponential decay constant. The *t*_{1/2} was evaluated from the data collected at 227 nm (Figure 8A), where CD spectra of both FGF10 protein variants showed the ellipticity maxima (Figure 8B). FGF10-STAB clearly outperformed FGF10-wt, showing *t*_{1/2} of its secondary structure of >24 hr at 37°C compare to FGF10-wt exhibiting *t*_{1/2} of 11.6 hr (Figure 8A). This example demonstrates that the decline of the biological activity of wild-type FGF10 corresponds with the loss of its secondary structure after heat-preincubation at 37°C, while FGF10-STAB retained full biological activity after one day of incubation at 37°C due to its preserved secondary structure.

### EXAMPLE 10. Long-term biological activity testing of 4-point FGF10 mutant, FGF10-STAB, using the MCF7 cell reporter assay

The ability of FGF10-STAB to activate prolonged FGFR signaling was tested by fluorescent reporter assay using MCF7 cells transfected by TR01G vector carrying the pKrox24(MapERK)-DsRed reporter. The pKrox24(MapERK)-DsRed responded to the activation of FGFR signaling upon addition of FGF10 into the cell culture, allowing sensitive monitoring of the FGF10 ability to stimulate FGFR/ERK MAP signaling pathway. For reporter assay, 50,000 MCF7 cells expressing DsRed (Discosoma Red Fluorescent Protein) under the control of pKrox24(MapERK) promoter were plated into 24 well plates in 0.5 mL of complete medium. The next day, the cells were stimulated with FGF10 at final concentration of 100 ng/mL for 24 hr. The expression of DsRed was monitored by automatic incubation microscope BioStation CT (Nikon) over the entire 24 hr period of time and plotted against signal recorded for control represented by FGF10-free cells transfected with pKrox24(MapERK)-DsRed. For the cell treatment, FGF10 was diluted at concentration of 100 ng/mL in complete medium, and pre-incubated at 37°C for 2 hours.

The cell treatment with recombinant FGF10-wt resulted in transcriptional induction of the pKrox24 reporter, which responds to activation of the FGFR/ERK MAP kinase pathway. The FGF-mediated pKrox24 induction was monitored for 24 hours, and was approximately 2.5 times stronger in cells treated with FGF10-STAB, compared to FGF10-wt (Figure 9A). When the FGF10 was preincubated in complete media for 2 hours prior to addition to cells, almost none pKrox24 induction was found with FGF10-wt, compared to normal activation caused by FGF10-STAB (Figure 9B). This example demonstrates that FGF10-STAB is capable of sustained activation of the FGFR signaling, resistant to the unfolding and proteolytic degradation in the presence of serum.

### EXAMPLE 11. Thermostability determination of 4-point FGF10 mutant using 4MBr-5 cells

The ability of FGF10-STAB to promote proliferation of 4MBr-5 cells after the exposure to 37°C up to 7 days was further tested to more explore the protein stability. 4MBr-5 cells were seeded in 96-well plates at 7.5 × 10³ cells per well in 150 µL of 4MBr-5 starvation media (Ham's F-12K - Gibco, 10% Newborn Calf Serum, 1% PenicillinG/Streptomycin Sulfate, 2 µg/mL heparin). Protein treatment was administered to the cells 3 hours after seeding, to allow proper adhesion of cells. Prior the testing, both FGF10-STAB and FGF10-wt at the concentration 8 µg/mL were incubated at 37°C for 1 day, 3 days and 7 days. After the preincubation, the protein solutions were serially diluted in starvation media and 50 µL of protein solution in a final concentration ranging from 0.001 ng/mL to 2000 ng/mL was added to the wells. Each protein concentration was tested in triplicate. Mouse EGF was used as a positive control. Following 3 days of cultivation, 20 µL of resazurin solution were added to each well and the plates were incubated overnight. Measurement of metabolic activity was performed by fluorescence reading (560 nm excitation, 590 nm emission) on Synergy 4H reader. Preincubation of FGF10-STAB at 37°C for 24 hr, 3 and 7 days did not affect the protein ability to effectively induce proliferation of 4MBr-5 cells (Figure 10). Determined ED₅₀ values for FGF10-STAB after its preincubation at 37°C for the three tested time periods were < 15 ng/mL, which correspond to the ED₅₀ value of the protein without preincubation (ED₅₀ = 0.37 ng/mL). On the other hand, treatment of FGF10-wt at 37°C for 24 hr, 3 and 7 days led to a gradual decrease of the protein activity, demonstrated by increased ED₅₀ values (Figure 10 and Table 4). This example demonstrates that FGF10-STAB exhibits unmodified biological activity even after 7days preincubation at 37°C.

**Table 4. Comparison of biological activity of FGF10-wt and FGF10-STAB without preincubation and after preincubation at 37°C for 24 hr, 3 and 7 days.**

| | **ED₅₀ (ng/mL) determined using 4MBr-5 cells** | |
|---|---|---|
| **Preincubation conditions** | **FGF10-wt** | **FGF10-STAB** |
| No preincubation (control) | 0.95 | 0.37 |
| 37°C for 1 day | 12.5 | 1.0 |
| 37°C for 3 days | 21.1 | 0.26 |
| 37°C for 7 days | 34.8 | 11.1 |

| | | |
|---|---|---|
| ED₅₀: median effective dose. | | |

### EXAMPLE 12. Thermostabilized 4-point FGF10 mutant exhibits improved resistance towards proteolytic degradation

To characterize resistance of FGF10-STAB against the proteolytic degradation, 1 mg/mL of FGF10-STAB and FGF10-wt were incubated at 37°C for 15 minutes in 20 mM phosphate buffer containing 750 mM sodium chloride. Trypsin (in a final concentration of 2 µg protease/100 µg FGF10) was then added to the protein solution and the digestion mixture was incubated at 37°C. Aliquots were removed at selected times, added to an equal volume of electrophoresis diluent, heated at 95°C for 5 min and analyzed by SDS-polyacrylamide gel electrophoresis. The protein bands were analyzed using densitometric analysis. The progressive degradation of FGF10-wt was observed with no protein remaining already after 10 minutes of incubation with trypsin. In contrast, there was no evidence of significant proteolytic degradation at 10 minutes and the band of FGF10-STAB with diminishing intensity in time was detectable even after 180 minutes of incubation with trypsin (Figure 11). This example demonstrates enhanced resistence of FGF10-STAB towards proteolytic degradation when compared to FGF10-wt.

### EXAMPLE 13. N-terminal truncation of 31 amino acids from the mature form of human FGF10 polypeptide resulting in the amino acid sequence Ser69-Ser208 of SEQ ID NO: 1 does not negatively affect FGF10 stability and activity

The FGF10 polypeptide consisting of the amino acid sequence Ser69-Ser208 of SEQ ID NO: 1 and its stable variant carrying the substitution L152F was constructed and characterized in order to demonstrate that N-terminal truncation of 31 amino acids from the mature form of human FGF10 polypeptide do not affect the protein stability and activity. The FGF10 polypeptide consisting of amino acid sequence Ser69 - Ser208 (FGF10⁶⁹⁻²⁰⁸) with N-terminal Met was constructed by side-directed mutagenesis using Q5^{®} Site-Directed Mutagenesis Kit (New England Biolabs) according to the instructions of the manufacturer. Mutagenesis was done by whole plasmid PCR using the plasmid pET28b-His-thrombin::*fgf10* as a template and two oligonucleotides (Fw: AGCTATAATCATCTGCAGGGTGATGTTCG, Rv: CATATGGCTGCCGCGCGG) which were designed using NEBaseChanger^{™} tool. Subsequently, the single point substitution L152F (numbering of the mutation corresponds to SEQ ID NO: 1) was introduced into the FGF10⁶⁹⁻²⁰⁸ polypeptide by whole plasmid PCR using the vector pET28b-His-thrombin::*fgf10⁶⁹⁻²⁰⁸* as a template and two non-overlapping oligonucleotides carrying the desired mutation. Resulting constructs were transformed into *E*. *coli* NEB5-alpha competent cells. Cells were plated on agar plates with kanamycin (50 µg/mL) and grown overnight at 37°C. Plasmids were isolated and nucleotide sequences were confirmed by commercial sequencing. *E. coli* BL21 (DE3) cells were transformed with expression vectors, plated on agar plates with kanamycin and grown overnight at 37°C. Single colonies were used to inoculate 10 mL of LB medium with kanamycin (50 µg/mL) and cells were grown overnight at 37°C. The overnight cultures were used to inoculate 1 L of LB medium with kanamycin. Cells were cultivated at 37°C. The expression was induced with IPTG to a final concentration of 0.5 mM. Cells were then cultivated overnight at 20°C. At the end of cultivation, biomass was harvested by centrifugation, washed with purification buffer (20 mM di-potassium hydrogen phosphate and potassium dihydrogen phosphate, pH 7.5, 0,5 M NaCl, 10 mM imidazole) and wet biomass was frozen at -80°C. Defrosted cultures were disrupted by sonication and lysate was centrifuged. Proteins were purified from crude extracts using single step nickel affinity chromatography. Purified proteins were dialyzed against buffer containing 750 mM NaCl. The protein purity was proved by SDS-PAGE in 15% polyacrylamide gel (Figure 12). Correct folding and thermostability of FGF10 polypeptides were determined by CD spectroscopy. CD spectra were collected from 200 to 260 nm, at 100 nm per min, 1 s response time and 2 nm bandwidth using Chirascan CD spectrometer. CD spectra of FGF10⁶⁹⁻²⁰⁸ polypeptide and its variant FGF10⁶⁹⁻²⁰⁸+L152F were comparable with the CD spectra of recombinant human FGF10 (FGF10-wt) and its variant comprising the same stabilizing amino acids substitution L152F, implying that N-terminal truncation does not negatively affect the folding of FGF10 polypeptide (Figure 13). The thermal unfolding of 0.2 mg/mL protein solutions in 20 mM phosphate buffer pH 7.5 containing 750 mM sodium chloride was followed by monitoring the ellipticity at 227 nm over the temperature range from 25 to 90°C at a heating rate 1 °C/min using Chirascan CD spectrometer. The melting temperatures (*T*ₘ) were evaluated as a midpoint of normalized ellipticity thermal transitions. N-terminal truncation has neither negative nor positive effect on thermal stability of FGF10 polypeptide, since FGF10⁶⁹⁻²⁰⁸ exhibits comparable *T*ₘ (52.7°C) as FGF10-wt (*T*ₘ 53.0°C). Introduction of the stabilizing amino acid substitution L152F to both FGF10 polypeptides, FGF10-wt and FGF10⁶⁹⁻²⁰⁸ increase consistently their stability by 7°C.

Biological activity of FGF10⁶⁹⁻²⁰⁸ polypeptide and its variant FGF10⁶⁹⁻²⁰⁸+L152F was determined by cell proliferation assay using 4MBr-5 cell system (Rubin, J.S. et al., 1989, Proc Natl Acad Sci U S A, 86:802-806). 4MBr-5 cells were seeded in 96-well plates at 7.5 × 10³ cells per well in 150 µL of 4MBr-5 starvation media (Ham's F-12K - Gibco, 10% Newborn Calf Serum, 1% PenicillinG/Streptomycin Sulfate, 2 µg/mL heparin). Protein treatment was administered to the cells 3 hours after seeding, final protein concentrations ranged from 0.001 ng/mL to 2000 ng/mL. Cell were cultivated for additional 3 days after the treatment. Cell proliferation was measured using resazurin fluorescence assay. Experiments were performed in triplicates. Both FGF10⁶⁹⁻²⁰⁸ and FGF10⁶⁹⁻²⁰⁸+L152F exhibits comparable ability to promote proliferation of 4MBr-5 cells as FGF10-wt (Figure 14), with determined ED₅₀ values 0.5 and 1.9 ng/mL, respectively, corresponding to the expected ED₅₀ value for this effect < 15 ng/mL.

### EXAMPLE 14. Change of 12% of the amino acid sequence of thermostable FGF10 can be achieved without loss of the protein thermostability and activity

Gene encoding FGF10 polypeptide having 88% sequence identity to SEQ ID NO: 3 or SEQ ID NO: 5, which corresponds to FGF10 from *Falco peregrinus* (FGF10-Fp), was artificially synthesized and subcloned in the *Ndel* and *Xhol* sites of the expression vector pET28b-His-thrombin under the control of the T7lac promoter. Subsequently, the single point substitution L152F (numbering of the mutation corresponds to SEQ ID NO: 1) was introduced into the FGF10-Fp polypeptide having 88% sequence identity to SEQ ID NO: 3 or SEQ ID NO: 5 by whole plasmid PCR using the vector pET28b-His-thrombin::*fgf10-fp* as a template and two non-overlapping oligonucleotides carrying desired mutation. Resulting constructs were transformed into *E. coli* NEB5-alpha competent cells. The cells were plated on agar plates with kanamycin (50 µg/mL) and grown overnight at 37°C. Plasmids were isolated and nucleotide sequences were confirmed by commercial sequencing. *E. coli* BL21(DE3) cells were transformed with the expression vectors, plated on agar plates with kanamycin and grown overnight at 37°C. Single colonies were used to inoculate 10 ml of LB medium with kanamycin (50 µg/mL) and cells were grown overnight at 37°C. The overnight cultures were used to inoculate 1 L of LB medium with kanamycin. Cells were cultivated at 37°C. The expression was induced with IPTG to a final concentration of 0.5 mM. Cells were then cultivated overnight at 20°C. At the end of cultivation, biomass was harvested by centrifugation, washed with purification buffer (20 mM di-potassium hydrogen phosphate and potassium dihydrogen phosphate, pH 7.5, 0.5 M NaCl, 10 mM imidazole) and wet biomass was frozen at -80°C. Defrosted cultures were disrupted by sonication and lysate was centrifuged. Proteins were purified from crude extracts using single-step nickel affinity chromatography. Purified proteins were dialyzed against a buffer containing 750 mM NaCl. The protein purity was proved by SDS-PAGE in 15% polyacrylamide gel (Figure 15). Correct folding and thermostability of FGF10 polypeptides were determined by CD spectroscopy. CD spectra were collected from 200 to 260 nm, at 100 nm per min, 1 s response time and 2 nm bandwidth using Chirascan CD spectrometer. CD spectra of FGF10 polypeptide having 88% sequence identity to SEQ ID NO: 3 or SEQ ID NO: 5, as well as its single point variant, comprising the amino acids substitution L152F, were comparable with the CD spectra of recombinant human FGF10-wt and its variant comprising the same amino acids substitution L152F (Figure 16), implying correct folding of newly constructed FGF10 polypeptides with introduced 12% of amino acid sequence variability. The thermal unfolding of 0.2 mg/mL protein solutions in 20 mM phosphate buffer pH 7.5 containing 750 mM sodium chloride was followed by monitoring the ellipticity at 227 nm over the temperature range from 25 to 90°C at a heating rate 1 °C/min using Chirascan CD spectrometer. The melting temperatures (*T*ₘ) were evaluated as a midpoint of normalized ellipticity thermal transitions. Recombinant falcon FGF10 polypeptide (FGF10-Fp) having 88% sequence identity to SEQ ID NO: 3 or SEQ ID NO: 5 exhibited 5°C higher melting temperature than recombinant human FGF10 (FGF10-wt). The observed difference in the melting temperature of FGF10 polypeptides could be attributed to the fact that core body temperature of the birds is typically 3-6°C higher than the body temperature of human (McCafferty, D.J., et al., 2015, Anim. Biotelemetry 3:33). However, the introduction of the same stabilizing mutation L152F to both FGF10-wt and FGF10-Fp polypeptides enhanced their melting temperatures by 7 and 5°C, respectively.

Biological activity of FGF10-Fp and its variant carrying the single-point substitution L152F (FGF10-Fp+L152F) was determined by cell proliferation assay using 4MBr-5 cell system (Rubin, J.S. et al., 1989, Proc Natl Acad Sci U S A, 86:802-806). 4MBr-5 cells were seeded in 96-well plates at 7.5 × 10³ cells per well in 150 µL of 4MBr-5 starvation media (Ham's F-12K - Gibco, 10% Newborn Calf Serum, 1% PenicillinG/Streptomycin Sulfate, 2 µg/mL heparin). Protein treatment was administered to the cells 3 hours after seeding, final protein concentrations ranged from 0.001 ng/mL to 2000 ng/mL. Cell were cultivated for additional 3 days after the treatment. Cell proliferation was measured using resazurin fluorescence assay. Experiments were performed in triplicates. The ED₅₀ values for FGF10-Fp and FGF10-Fp+L152F determined in proliferation assay of 4MBr-5 were 12.7 and 6.1 ng/mL, respectively (Figure 17). This example demonstrates that the introduction of 12% of amino acid sequence variability can be achieved without significant loss of stability of thermostable FGF10 polypeptides while preserving their biological activity.

### EXAMPLE 15. Presence of FGF10-STAB in culture medium enhance lungosphere and lung organoid formation

FGF10 has an important role during lung development, differentiation and regeneration. Therefore, the effect of FGF10-STAB on lungospheres and lung organoids formation, branching and differentiation was investigated. Lung epithelial cells were isolated as described previously (Rabata, A. et al., 2017, Methods Mol Biol, 1612:149-165) and cultured in non-adherent conditions. Unsorted lung epithelial cells were seeded in polyHEMA-treated six-well plates at 2.5 × 10⁴ to 5 × 10⁴ cells in 2 mL/well of a lungosphere medium (1 × B-27 without vitamin A, 100 U/mL penicillin, 100 µg/mL streptomycin, 4 µg/mL heparin, 20 ng/mL murine EGF, 10 ng/mL FGF10-wt or FGF10-STAB, 10 µM Y-27632 in phenol red-free DMEM/F12). The plates were incubated in a humidified atmosphere at 37°C, 5% CO₂. A fresh lungosphere medium (without Y-27632) was added every 3 days. Lungospheres were counted after 10-15 days of culture. Lungosphere forming efficiency was calculated as a ratio of number of lungospheres formed and number of cells seeded.

For lung organoid cultivation in 3D Matrigel, the lung epithelial cells were resuspended in Matrigel and plated into Matrigel-coated 24-well plate in domes (1.0-1.5 × 10⁴ cells in 50 µL Matrigel/well). The plate was incubated at 37°C for 45 min before adding 1 mL of medium (1 × B-27 without vitamin A, 100 U/mL penicillin, 100 µg/mL streptomycin, 20 ng/mL murine EGF, 2 nM FGF10-wt or FGF10-STAB, 10 µM Y-27632 in phenol red-free DMEM/F12) per well. The cells were incubated in a humidified atmosphere at 37°C, 5% CO₂. Medium was changed every 3 days. Lung organoids were counted after 21 days of culture. Organoid forming efficiency was calculated as a ratio of number of lung organoids formed and number of cells seeded.

Obtained results revealed higher lungosphere and organoid forming efficiency observed with FGF10-STAB than FGF10-wt (Figure 18). Furthermore, the organoids formed with FGF10-STAB displayed more branching and higher ration of staining for airway and alveolar cell marker than for basal cell marker. This example demonstrates that the presence of FGF10-STAB in lungosphere/lung organoid medium enhance the lungosphere/organoid formation and differentiation.

## Claims

1. A thermostable FGF10 polypeptide possessing FGF10 activity and having or comprising at least 85% sequence identity of SEQ ID NO:3 that has an amino acid sequence from Ser69 to Ser208 of SEQ ID NO:1, or a fragment thereof, wherein the thermostable FGF10 polypeptide or the fragment thereof comprising at least an amino acid substitution L152F.

2. The thermostable FGF10 polypeptide according to Claim 1 wherein the thermostable FGF10 polypeptide has or comprises SEQ ID NO:3, or the fragment thereof, wherein the thermostable FGF10 polypeptide or the fragment thereof comprising at least an amino acid substitution L152F.

3. The thermostable FGF10 polypeptide according to Claim 1 or Claim 2 wherein the thermostable FGF10 polypeptide or the fragment thereof further comprises at least one amino acid substitutions selected from a group consisting of V123I, Q175E, and N181D.

4. The thermostable FGF10 polypeptide according to Claim 1 or Claim 2 wherein the thermostable FGF10 polypeptide or the fragment thereof further comprises at least two amino acid substitutions selected from a group consisting of V123I, Q175E, and N181D.

5. The thermostable FGF10 polypeptide according to any of Claims 1 to 2 wherein the thermostable FGF10 polypeptide or the fragment thereof further comprises three amino acid substitutions V123I, Q175E, and N181D.

6. The thermostable FGF10 polypeptide according to any of Claims 1 to 5, further carrying:
- at least part or all amino acids of SEQ ID NO:4 or methionine at the N-terminus of SEQ ID NO:3;
and/or
- at least one of a short fusion amino acid sequences (tag) up to a maximum length of 20 amino acids at the N-terminal or C-terminal parts.

7. A thermostable FGF10 polypeptide according to Claim 1 having or comprising at least 85% sequence identity of SEQ ID NO:5 that has an amino acid sequence from Leu40 to Ser208 of SEQ ID NO:1, or a fragment thereof, wherein the thermostable FGF10 polypeptide or the fragment thereof comprising at least an amino acid substitution L152F.

8. The thermostable FGF10 polypeptide according to claim 7 wherein the thermostable FGF10 polypeptide has or comprises SEQ ID NO:5, or the fragment thereof, wherein the thermostable FGF10 polypeptide or the fragment thereof comprising at least an amino acid substitution L152F.

9. The thermostable FGF10 polypeptide according to Claim 7 or 8, the thermostable FGF10 polypeptide or the fragment thereof further comprises at least one amino acid substitution selected from a group consisting of V123I, Q175E, and N181D.

10. The thermostable FGF10 polypeptide according to Claim 7 or 8, the thermostable FGF10 polypeptide or the fragment thereof further comprises at least two amino acid substitutions selected from a group consisting of V123I, Q175E, and N181D.

11. The thermostable FGF10 polypeptide according to any of Claims 7 to 8 wherein the thermostable FGF10 polypeptide or the fragment thereof further comprises three amino acid substitutions V123I, Q175E, and N181D.

12. The thermostable FGF10 polypeptide according to any of Claims 7 to 11 wherein it is selected from a group of the FGF10 polypeptides comprising SEQ ID NO:5, SEQ ID NO:7, SEQ ID NO:8, SEQ ID NO:9, SEQ ID NO:10, SEQ ID NO:11, SEQ ID NO:12 or SEQ ID NO:13.

13. The thermostable FGF10 polypeptide according to any of Claims 7 to 12, further carrying:
- at least part or all amino acids of SEQ ID NO:6 or methionine at the N-terminus of SEQ ID NO:5;
and/or
- at least one of a short fusion amino acid sequence (tag) up to a maximum length of 20 amino acids at the N-terminal or C-terminal parts.

14. A thermostable FGF10 polypeptide according to any of Claims 7 to 12 further carrying SEQ ID NO:6 at the N-terminus any of SEQ ID NO:5, SEQ ID NO:7, SEQ ID NO:8, SEQ ID NO:9, SEQ ID NO:10, SEQ ID NO:11, SEQ ID NO:12, or SEQ ID NO:13.

15. The thermostable FGF10 polypeptide according to any of Claims 1 to 14 for use in regenerative medicine, preferably for lung epithelial regeneration after various stresses or for curing of wounds and ulcers or other related medical applications, such as cardiac treatment.

16. Use the thermostable FGF10 polypeptide according to any of Claims 1 to 14 in cosmetics, preferably support of collagen synthesis, skin strength, anti-aging treatment, and non-therapeutic stimulation of hair growth.

17. A culture medium for proliferation and differentiation of the human embryonic stem cells, or formation and differentiation of spheroids and organoids, preferably lung organoids, stomach organoids, liver organoids, pancreas organoids, and prostate organoids comprising an effective amount of the thermostable FGF10 polypeptide according to any of Claims 1 to 14 in the range of 10 ng/mL to 500 ng/mL of culture medium.
